(19) Europäisches Patentamt
European Patent Office
Office européen des brevets

(11) Veröffentlichungsnummer: **0 084 756**
**A1**

(12) **EUROPÄISCHE PATENTANMELDUNG**

(21) Anmeldenummer: **82810027.1**

(22) Anmeldetag: **22.01.82**

(51) Int. Cl.³: **C 07 D 413/04**, A 61 K 31/44
// C07D213/50, C07D213/53

(43) Veröffentlichungstag der Anmeldung: **03.08.83**
**Patentblatt 83/31**

(84) Benannte Vertragsstaaten: **BE DE FR IT LU NL SE**

(71) Anmelder: **CIBA-GEIGY AG, Patentabteilung Postfach, CH-4002 Basel (CH)**

(72) Erfinder: **Sallmann, Alfred, Dr., Joachimsackerstrasse 12, CH-4103 Bottmingen (CH)**

(54) **Trisubstituierte Oxazaverbindungen.**

(57) Die Verbindung betrifft neue trisubstituierte Oxazaverbindungen, insbesondere Verbindungen der allgemeinen Formel

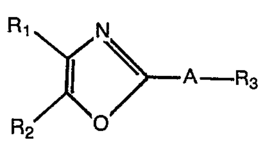

(I),

worin einer der Reste $R_1$ und $R_2$ Heteroaryl und der andere carbocyclisches Aryl oder Heteroaryl bedeutet und A einen zweiwertigen Kohlenwasserstoffrest darstellt und $R_3$ Carboxy, verestertes oder amidiertes Carboxy bedeutet, ihre Isomeren und ihre Salze.

Die Verbindungen der Formel (I), welche als Hauptphlogistatika verwendet werden können, werden nach an sich bekannten Verfahren hergestellt.

EP 0 084 756 A1

CIBA-GEIGY AG
Basel (Schweiz)

Case 4-13769/12985

# Trisubstituierte Oxazaverbindungen

Die Erfindung betrifft neue trisubstituierte Oxazaverbindungen, insbesondere Verbindungen der allgemeinen Formel

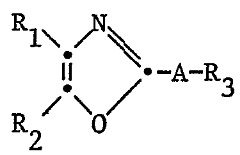

(I) ,

worin einer der Reste $R_1$ und $R_2$ Heteroaryl und der andere carbocyclisches Aryl oder Heteroaryl bedeutet und A einen zweiwertigen Kohlenwasserstoffrest darstellt und $R_3$ Carboxy, verestertes oder amidiertes Carboxy bedeutet, ihre Isomeren und ihre Salze, Verfahren zu ihrer Herstellung, solche Verbindungen enthaltende pharmazeutische Präparate sowie die Verwendung von Verbindungen der Formel (I), ihrer Isomeren und ihrer Salze als Arzneimittelwirkstoffe und/oder zur Herstellung pharmazeutischer Präparate.

Carbocyclisches Aryl ist beispielsweise monocyclisches carbocyclisches Aryl, wie gegebenenfalls substituiertes Phenyl.

Heteroaryl ist beispielsweise monocyclisches, vorzugsweise 5- oder 6-gliedriges Heteroaryl, wobei mindestens ein Ringglied ein Heteroatom, wie ein Stickstoff-, Sauerstoff- oder Schwefelatom, darstellt, wobei ein Stickstoffatom auch gegebenenfalls in oxidierter Form vorliegen kann. Solche 5-gliedrigen Reste sind z.B. Pyrrolyl, wie 2-Pyrrolyl.

Als 6-gliedriges Heteroaryl kommt z.B. Pyridyl, wie 2-, 3- oder 4-Pyridyl, 1-Oxidopyridyl, wie 1-Oxido-3-pyridyl oder 1-Oxido-4-pyridyl, und Pyrimidyl, wie 2-Pyrimidyl, in Frage.

Als Substituenten von carbocyclischem Aryl, wie Phenyl, bzw. Heteroaryl, wie Pyridyl oder 1-Oxido-pyridyl, kommen beispielsweise Halogen, Niederalkyl, Hydroxy, Niederalkoxy und/oder Acyloxy in Betracht. Acyloxy ist beispielsweise von einer organischen Carbonsäure abgeleitet und bedeutet z.B. Niederalkanoyloxy.

Ein Kohlenwasserstoffrest A ist beispielsweise ein zweiwertiger aliphatischer, cycloaliphatischer oder cycloaliphatisch-aliphatischer Kohlenwasserstoffrest.

Als zweiwertige aliphatische Kohlenwasserstoffreste kommen beispielsweise Niederalkylen, Niederalkyliden, Niederalkenylen oder Niederalkenyliden in Frage. Zweiwertige cycloaliphatische Kohlenwasserstoffe sind beispielsweise monocyclische 3- bis 8-gliedrige Cycloalkylene oder Cycloalkylidene. Cycloaliphatisch-aliphatische Kohlenwasserstoffreste sind beispielsweise solche, die als cycloaliphatischen Rest einen monocyclischen 3- bis 8-gliedrigen cycloaliphatischen Rest und als aliphatischen Rest Niederalkyliden aufweisen, wie Cycloalkyl-niederalkyliden.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, Propanol, Isopropanol, n-Butanol, sec- oder tert.-Butanol, während als cycloaliphatischer Alkohol beispielsweise ein 3- bis 8-gliedriges Cycloalkanol, wie Cyclopentanol,-hexanol oder -heptanol, in Frage kommt. Als Substituenten solcher Niederalkanole bzw. Cycloalkanole kommen beispielsweise Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Hydroxy-hydroxyniederalkoxyniederalkoxy, Niederalkoxyniederalkoxyniederalkoxy, gegebenenfalls im Phenylteil substituiertes Phenylniederalkoxyniederalkoxyniederalkoxy.

Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy in Betracht. Ein aromatischer Alkohol ist beispielsweise ein Phenol oder heterocyclischer Alkohol, welche jeweils gegebenenfalls durch Niederalkyl, Niederalkoxy, Halogen und/oder Trifluormethyl substituiert sein können, insbesondere Hydroxypyridin, z.B. 2-, 3- oder 4-Hydroxypyridin.

Amidiertes Carboxy ist beispielsweise Carbamoyl, durch Hydroxy, Amino, oder gegebenenfalls substituiertes Phenyl monosubstituiertes, durch Niederalkyl mono- oder disubstituiertes oder durch 4- bis 7-gliedriges Alkylen bzw. 3-Aza-, 3-Niederalkylaza-, 3-Oxo- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- oder N,N-Dipropylcarbamoyl, Pyrrolidino- oder Piperidinocarbonyl, Morpholino-, Piperazino- bzw. 4-Methylpiperazino- sowie Thiomorpholinocarbonyl, Anilinocarbonyl oder durch Niederalkyl, Niederalkoxy und/oder Halogen substituiertes Anilinocarbonyl zu nennen.

In der vorliegenden Beschreibung sind unter "niederen" organischen Resten und Verbindungen vorzugsweise solche zu verstehen, die bis und mit 7, vor allem bis und mit 4 Kohlenstoffatome (C-Atome) enthalten.

Die vor- und nachstehend verwendeten Allgemeindefinitionen haben im Rahmen der vorliegenden Anmeldung in erster Linie die folgenden Bedeutungen:

Halogen ist z.B. Halogen bis und mit Atomnummer 35, wie Fluor, Chlor oder Brom, ferner Iod.

Niederalkyl ist z.B. Methyl, Ethyl, n-Propyl, Isopropyl, n-Butyl, Isobutyl, sec.-Butyl, tert.-Butyl, ferner ein Pentyl-, Hexyl-oder Heptylrest.

Niederalkoxy ist z.B. Methoxy, Ethoxy, n-Propyloxy, Isopropyloxy, n-Butyloxy, Isobutyloxy, sec.-Butyloxy oder tert.-Butyloxy.

Niederalkylthio ist z.B. Methyl-, Ethyl-, n-Propyl-, Isopropyl-, n-Butyl-, Isobutyl-, sec.-Butyl oder tert.-Butylthio.

Phenylniederalkoxy ist z.B. Phenylmethoxy, Phenylethoxy oder Phenylpropyloxy.

Phenylniederalkylthio ist z.B. Benzyl-, Phenylethyl- oder Phenylpropylthio.

Hydroxyniederalkoxy ist z.B. Hydroxyethoxy, Hydroxypropyloxy oder 1,2-Dihydroxypropyloxy.

Niederalkoxyniederalkoxy ist z.B. Methoxyethoxy, Ethoxyethoxy, Methoxypropyloxy oder Methoxybutyloxy.

Phenylniederalkoxyniederalkoxy ist z.B. 2-Benzyloxyethoxy oder 2-(2-Phenylethoxy)-ethoxy.

Niederalkanoyloxy ist z.B. Acetyl-, Propionyl-, Butyryl-, Iso-, sec- oder tert.-Butyryloxy.

Niederalkylen ist z.B. geradkettig, wie Methylen, Ethylen, 1,3-Propylen oder 1,4-Butylen, oder verzweigt, wie 1,2-Propylen, 1,2- oder 1,3-(2-Methyl)-propylen oder 1,2-Butylen.

Niederalkyliden weist ein tertiäres oder insbesondere ein quartäres C-Atom auf und ist z.B. Ethyliden oder 1,1- oder 2,2-Propyliden, ferner 1,1- oder 2,2-Butyliden oder 1,1-, 2,2- oder 3,3-Pentyliden.

Niederalkenylen ist z.B. Ethenylen, 1,2- oder 1,3-Propenylen oder 1,2-, 1,3- oder 1,4-Buten-2-ylen.

Niederalkenyliden ist z.B. Ethenyliden, 1,1-Propen-1-yliden, 1,1-Propen-2-yliden, ferner ein Butenyliden, wie 1,1-Buten-3-yliden.

Cycloalkylen ist z.B. Cyclopropylen, 1,2- oder 1,3-Cyclobutylen, 1,2-, 1,3- oder 1,4-Cyclopentylen, ferner ein Cyclohexylen.

Cycloalkyliden ist z.B. Cyclopropyliden, Cyclobutyliden, Cyclopentyliden oder Cyclohexyliden.

Cycloalkyl-niederalkyliden ist z.B. ein Cyclopropyl-, Cyclobutyl-, Cyclopentyl- oder Cyclohexyl-methylen , -ethyliden oder -propyliden, ferner ein Cyclohexyl-butyliden.

Carboxyniederalkoxy ist z.B. Carboxymethoxy, 2-Carboxyethoxy, 2-, 3-Carboxypropyloxy, 1-Carboxy-2-propyloxy, 2-, 3- oder 4-Carboxy-n-butyloxy, 1-Carboxy-2-methyl-propyl-3-oxy oder 1-Carboxy-2-methyl-propyl-2-oxy.

Niederalkoxycarbonyl-niederalkoxy enthält jeweils im Niederalkoxyteil unabhängig voneinander die vorstehend unter Niederalkoxy aufgeführten Bedeutungen.

Salze von erfindungsgemässen Verbindungen der Formel I sind vorzugsweise pharmazeutisch verwendbare Salze, wie pharmazeutisch verwendbare Säureadditionssalze, und/oder, wenn $R_3$ Carboxy bedeutet, innere Salze oder Salze mit Basen. Geeignete Säureadditionssalze sind beispielsweise Salze mit anorganischen Säuren, wie Mineralsäure, mit Sulfaminsäuren, wie Cyclohexylsulfaminsäure, mit organischen Carbonsäuren, wie Niederalkancarbonsäuren, gegebenenfalls ungesättigte Dicarbonsäuren, mit durch Hydroxy und/oder Oxo substituierten Carbonsäuren oder mit Sulfonsäuren, beispielsweise Sulfate oder Hydrohalogenide, wie Hydrobromide oder Hydrochloride, Oxalate, Malonate, Fumarate oder Maleinate, Tartrate, Pyruvate oder Citrate, Sulfonate, wie Methan-, Benzol- oder p-Toluolsulfonate.

Geeignete Salze mit Basen sind beispielsweise Alkali- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder von substituierten organischen Aminen, wie cyclische Amine, z.B. Morpholin, Thiomorpholin, Piperidin, Pyrrolidin, wie Mono-, Di- bzw. Triniederalkylamine oder Mono-, Di- bzw. Trihydroxyniederalkylamine , z.B. Mono-, Di- bzw. Triethanolamin. Mononiederalkylamine sind beispielsweise Ethyl- oder tert.-Butylamin. Diniederalkylamine sind z.B. Diethyl- oder Diisopropylamin, und als Triniederalkylamin kommt z.B. Triethylamin in Betracht.

Die neuen Verbindungen der Formel I und ihre pharmazeutisch verwendbaren Salze weisen wertvolle pharmakologische Eigenschaften auf. Insbesondere besitzen sie, z.B. bei lokaler Anwendung, eine ausgeprägte antiinflammatorische Wirkung.

Diese Eigenschaft lässt sich beispielsweise nach der von G. Tonelli, L. Thibault, Endocrinology 77, 625 (1965) entwickelten Methode durch Hemmung des mit Crotonöl induzierten Rattenohroedems bei der Normalratte im Dosisbereich von etwa 1 bis etwa 100 mg/ml nachweisen. So wurde beispielsweise für die Verbindung 2-[4-Phenyl-5-(3-pyridyl)-imidazol-2-yl]-essigsäureethylester im vorstehend beschriebenen Test ein $ED_{50}$-Wert von 17 mg/ml ermittelt.

Die erfindungsgemässen Verbindungen der Formel I sind daher als Arzneimittel, insbesondere externe (topische) Hautphlogistika für die Behandlung entzündlicher Dermatosen jeglicher Genese, wie bei leichten Hautirridationen, Kontaktdermatitis, Exanthemen, Verbrennungen, sowie als Schleimhautphlogistika für die Behandlung von Mukosaentzündungen, z.B. der Augen, Nase, Lippen, Genital-, Analregion, geeignet. Ferner können die Verbindungen als Sonnenschutzmittel verwendet werden.

Die Erfindung betrifft z.B. Verbindungen der Formel I, worin einer der Reste $R_1$ und $R_2$ einen 5- oder 6-gliedrigen, als Heteroatom(e) Stickstoff aufweisenden Heteroaryl bedeutet, welcher unsubstituiert oder durch

Halogen, Niederalkyl, Hydroxy, Niederalkoxy und/oder Niederalkanoyloxy substituiert sein kann, und der andere unsubstituiertes oder durch Halogen, Niederalkyl, Hydroxy, Niederalkoxy und/oder Niederalkanoyl substituiertes Phenyl oder einen 5- oder 6-gliedrigen, als Heteroatom(e) Stickstoff aufweisenden Heteroarylrest darstellt, der unsubstituiert oder durch Halogen, Niederalkyl, Hydroxy, Niederalkoxy und/oder Niederalkanoyl substituiert sein kann, bedeutet, A Niederalkylen, Niederalkyliden, Niederalkenylen, Niederalkenyliden, Cycloalkylen, Cycloalkyliden oder Cycloalkyl-niederalkyliden darstellt und $R_3$ Carboxy, mit einem Niederalkanol, 3- bis 8-gliedrigem Cycloalkanol, Phenol, einem Hydroxypyridin, einem substituierten Phenol bzw. Hydroxypyridin verestertes Carboxy, Carbamoyl oder durch Hydroxy, Amino, Phenyl oder substituiertes Phenyl mono-substituiertes, durch Niederalkyl mono- oder disubstituiertes oder durch 4- bis 7-gliedriges Alkylen bzw. 3-Aza-, 3-Niederalkylaza-, 3-Oxa- oder 3-Thiaalkylen disubstituiertes Carbamoyl bedeutet, wobei ein Niederalkanol unsubstituiert oder durch Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Hydroxyniederalkoxyniederalkoxy, Niederalkoxyniederalkoxyniederalkoxy, gegebenenfalls im Phenylteil substituiertes Phenylniederalkoxyniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy substituiert sein kann und substituiertes Phenyl, Phenol bzw. Hydroxypyridin jeweils durch Niederalkyl, Niederalkoxy, Halogen und/oder Trifluormethyl substituiert sein kann, ihre Isomeren sowie ihre Salze, insbesondere pharmazeutisch verwendbare Salze.

Die Erfindung betrifft beispielsweise Verbindungen der Formel I, worin einer der Reste $R_1$ und $R_2$ Pyridyl oder 1-Oxido-pyridyl, bedeutet, die unsubstituiert und/oder jeweils durch Halogen, Hydroxy, Niederalkyl, Niederalkoxy und/oder Niederalkanoyloxy substituiert sein können, und

der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, wie Methylen, Niederalkyliden mit bis und mit 7 C-Atomen, wie 2,2-Propyliden, Niederalkenylen mit bis und mit 4 C-Atomen, wie 1,3-Propen-2-ylen, Niederalkenyliden mit bis und mit 7 C-Atomen, wie 1,1-Buten-3-yliden, 3- bis 8-gliedriges Cycloalkylen, wie Cyclopropylen, 3- bis 8-gliedriges Cyclalkyliden, wie Cyclopentyliden, oder Cycloalkyl-niederalkyliden mit bis und mit 7 C-Atomen im Alkylidenteil und mit einem 3- bis 8-gliedrigen Cycloalkylteil, wie 2-Cyclohexyl-1,1-ethyliden, bedeutet und $R_3$ Carboxy, mit einem Niederalkanol, einem 3- bis 8-gliedrigen Cycloalkanol, Phenol oder einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Hydroxyniederalkoxyniederalkoxy, Niederalkoxyniederalkoxyniederalkoxy, gegebenenfalls im Phenylteil substituiertes Phenylniederalkoxyniederalkoxyniederalkoxy, Carboxyniederalkoxy, Niederalkoxycarbonylniederalkoxy, gegebenenfalls substituiertes Phenyl aufweisendes Niederalkoxycarbonyl-niederalkoxy oder Niederalkanoyloxy substituiert sein kann und substituiertes Phenol bzw. Phenyl jeweils durch Niederalkyl, Niederalkoxy, Halogen und/oder Trifluormethyl substituiert sein kann, ihre Isomeren sowie ihre Salze.

Die Erfindung betrifft beispielsweise Verbindungen der Formel I, worin einer der Reste $R_1$ und $R_2$ unsubstituiertes oder in zweiter Linie durch Halogen mit Atomnummer bis und mit 35, wie Chlor, Hydroxy, Niederalkyl mit bis und mit 4 C-Atomen, wie Methyl, und/oder Niederalkoxy mit bis

und mit 4 C-Atomen, wie Methoxy, substituiertes Phenyl bedeutet und der andere Pyridyl, wie 3-Pyridyl, oder 1-Oxido-pyridyl, wie 1-Oxido-3-pyridyl, bedeutet, die jeweils unsubstituiert oder in zweiter Linie durch Halogen mit Atomnummer bis und mit 35, wie Chlor, Hydroxy und/oder Niederalkoxy mit bis und mit 4 C-Atomen, wie Methoxy, substituiert sein können, A Niederalkylen mit bis und mit 4 C-Atomen, wie Methylen, Niederalkyliden mit bis und mit 7 C-Atomen, wie 2,2-Propyliden, Niederalkenyliden mit bis und mit 7 C-Atomen, wie 1,1-Buten-3-yliden, oder 3- bis 8-gliedriges Cycloniederalkyliden, wie 1,1-Cyclopentyliden, darstellen und $R_3$ Carboxy, Niederalkoxycarbonyl mit bis und mit 5 C-Atomen, wie Ethoxycarbonyl, wobei das Niederalkoxycarbonyl durch Niederalkanoyloxy mit bis und mit 5 C-Atomen, wie Pivaloyloxy, substituiert sein kann, Hydroxyniederalkoxy-niederalkoxycarbonyl, wie 2-(2-Hydroxyethoxy)-ethoxycarbonyl, Niederalkoxyniederalkoxy-niederalkoxycarbonyl, wie 2-(2-Methoxyethoxy)-ethoxycarbonyl, Hydroxyniederalkoxyniederalkoxy-niederalkoxycarbonyl, wie 2-[2-(2-Hydroxyethoxy)-ethoxy]-ethoxycarbonyl, oder Niederalkoxyniederalkoxyniederalkoxy-niederalkoxycarbonyl, wie 2-[2-(2-Methoxyethoxy)-ethoxy]-ethoxycarbonyl, jeweils mit bis und mit 4 C-Atomen im Niederalkoxy-Teil, bedeuten, ihre Isomeren sowie ihre Salze.

Die Erfindung betrifft in erster Linie Verbindungen der Formel I, worin einer der Reste $R_1$ und $R_2$ unsubstituiertes oder in zweiter Linie durch Halogen mit Atomnummer bis und mit 35, wie Chlor, Hydroxy oder Niederalkoxy mit bis und mit 4 C-Atomen, wie Methoxy, substituiertes Phenyl bedeutet und der andere Pyridyl, wie 3- oder 4-Pyridyl, oder 1-Oxido-pyridyl, wie 1-Oxido-3-pyridyl oder 1-Oxido-4-pyridyl, darstellt, A Niederalkyliden mit bis und mit 4 C-Atomen, wie 2,2-Propyliden, bedeutet, und $R_3$ Niederalkoxycarbonyl mit bis und mit 5 C-Atomen, wie Ethoxycarbonyl, darstellt, ihre Isomeren sowie ihre Salze.

Die Erfindung betrifft in erster Linie Verbindungen der Formel I, worin einer der Reste $R_1$ und $R_2$ unsubstituiertes oder in zweiter Linie durch Halogen mit Atomnummer bis und mit 35, wie Chlor, Hydroxy oder Niederalkoxy mit bis und mit 4 C-Atomen, wie Methoxy substituiertes Phenyl

bedeutet und der andere Pyridyl, wie 3- oder 4-Pyridyl, oder 1-Oxidopyridyl, wie 1-Oxido-3-pyridyl oder 1-Oxido-4-pyridyl, darstellt, A ein ein quartäres C-Atom aufweisendes Niederalkyliden mit bis und mit 4 C-Atomen, wie 2,2-Propyliden, wobei das quartäre C-Atom direkt an den Imidazolring gebunden ist, bedeutet, und $R_3$ Niederalkoxycarbonyl mit bis und mit 5 C-Atomen, wie Ethoxycarbonyl, darstellt, ihre Isomeren sowie ihre Salze.

Die Erfindung betrifft in allererster Linie Verbindungen der Formel I, worin $R_1$ Phenyl bedeutet, $R_2$ 1-Oxidopyridyl, wie 1-Oxido-3-pyridyl, darstellt, A 2,2-Propyliden bedeutet und $R_3$ Niederalkoxycarbonyl mit bis und mit 5 C-Atomen, wie Ethoxycarbonyl, darstellt, ihre Isomeren sowie ihre Salze.

Die Erfindung betrifft namentlich die in den Beispielen genannten neuen Verbindungen und ihre Salze, insbesondere pharmazeutisch verwendbare Salze von solchen Verbindungen mit salzbildenden Gruppen, sowie die in den Beispielen aufgeführten Herstellungsverfahren.

Eine Verfahrensweise zur Herstellung von Verbindungen der Formel (I) ist beispielsweise dadurch gekennzeichnet, dass man eine Verbindung der Formel

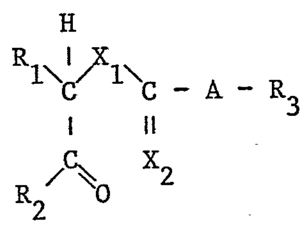

(II) ,

ein Tautomeres und/oder ein Salz davon, worin einer der Reste $X_1$ und $X_2$ gegebenenfalls substituiertes Imino und der andere Oxy oder gegebenenfalls substituiertes Imino bedeutet, cyclisiert oder worin $X_1$ Oxy und $X_2$ Oxo darstellen, unter Kondensation cyclisiert und gewünschtenfalls eine verfahrensgemäss erhätliche Verbindung der Formel (I) in eine andere Verbindung überführt, eine verfahrensgemäss erhältliche freie Verbindung in ein Salz oder ein verfahrensgemäss erhältliches Salz in ein anderes Salz

oder in eine freie Verbindung überführt und, wenn erwünscht, ein verfahrensgemäss erhältliches Isomerengemisch in seine Komponenten auftrennt.

Tautomere von Verbindungen der Formel (II) sind beispielsweise solche, bei denen eine partielle Enol- bzw. Enamin-Gruppierung in Form der entsprechenden tautomeren Keto- bzw. Ketiminform vorliegt und umgekehrt, wobei die jeweiligen Tautomeren miteinander im Gleichgewicht stehen.

Die Cyclisierung einer Verbindung der Formel II, einem Tautomeren und/oder Salz davon erfolgt in üblicher, insbesondere in der aus der Literatur für analoge Reaktionen bekannten Weise, erforderlichenfalls unter Erwärmen, wie in einem Temperaturbereich von etwa 20° bis etwa 250°C, unter Druck und/oder in Gegenwart eines katalytischen Mittels, vorzugsweise einer Säure oder einem Säureanhydrid. Als Säuren eignen sich beispielsweise anorganische Säuren, wie Mineralsäuren, z.B. Schwefelsäure, Polyphosphorsäure oder Halogenwasserstoffsäure, wie Chlorwasserstoffsäure oder organische Säuren, wie Niederalkancarbonsäuren, z.B. Essigsäure. Als Säureanhydride kommen z.B. Mineralsäureanhydride, wie Sulfuryl- oder Phosphor(oxy)halogenide, z.B. Phosphoroxychlorid, in Betracht. Dabei arbeitet man erforderlichenfalls in einem inerten Lösungsmittel, beispielsweise einem gegebenenfalls halogenierten Kohlenwasserstoff, wie Chloroform, Chlorbenzol, Hexan, Pyridin oder Toluol, einem Niederalkanol, wie Methanol, z.B. Dimethylformamid oder Formamid, oder einer Niederalkancarbonsäure, wie Ameisen- oder Essigsäure, und/oder unter Inertgas, wie Stickstoff.

Die Ausgangsstoffe der Formel II, ihre Tautomeren und/oder Salze werden nach an sich bekannten Verfahren zum überwiegenden Teil in situ gebildet und unter den Reaktionsbedingungen ohne Isolierung weiter zu der Verbindung der Formel I umgesetzt. Dabei kann die Cyclisierung im Anschluss an eine vorgelagerte Kondensation erfolgen.

So kann man in einer bevorzugten Ausführungsform des vorstehenden Verfahrens beispielsweise eine Verbindung der Formel

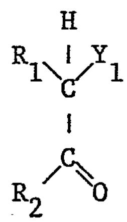 (IIa) ,

ein Tautomeres und/oder Salz davon, worin $Y_1$ gegebenenfalls funktionell abgewandeltes Hydroxy bedeutet, mit einer Verbindung der Formel $R_3$-A-$Y_2$ (IIb), worin $Y_2$ mindestens ein Stickstoffatom aufweisender funktionell abgewandeltes Carboxy darstellt, oder einem Salz davon oder eine Verbindung der Formel (IIa), ein Tautomeres und/oder Salz davon, worin $Y_1$ Amino bedeutet, mit einer Verbindung der Formel (IIb), worin $Y_2$ gegebenenfalls funktionell abgewandeltes Carboxy darstellt, oder einem Salz davon, gegebenenfalls in Gegenwart eines Kondensationsmittels, umsetzen. Dabei kann beispielsweise eine entsprechende Verbindung der Formel (II) oder ein Tautomeres davon gebildet werden, die unter den Reaktionsbedingungen erfindungsgemäss weiter reagiert.

In einer besonders bevorzugten Ausführungsform des vorstehend beschriebenen Verfahrens setzt man beispielsweise Verbindungen der Formel

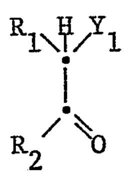 (IIa),

worin $Y_1$ gegebenenfalls reaktionsfähig verestertes Hydroxy bedeutet, mit Carbonsäuren der Formel $R_3$-A-COOH (IIc), deren Salze oder deren funktionelle Derivate und mit Ammoniak um, oder Verbindungen der Formel

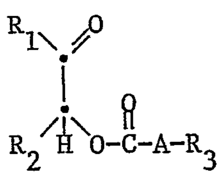 (IId),

welche durch Kondensation von Verbindungen der Formel (IIa) mit gegebenenfalls funktionellen Derivaten von Verbindungen der Formel (IIc) erhältlich sind, mit Ammoniak um.

Salze von Verbindungen der Formel (II) und (IIa) sind beispielsweise Säureadditionssalze, wie Hydrohalogenide. Funktionell abgewandeltes Hydroxy $Y_1$ ist beispielsweise Amino oder reaktionsfähiges verestertes Hydroxy, wie mit einer anorganischen Mineralsäure, z.B. Halogenwasserstoffsäure, oder mit einer organischen Sulfonsäure, z.B. gegebenenfalls substituierten Benzolsulfonsäure, verestertes Hydroxy. Als Beispiele für reaktionsfähiges verestertes Hydroxy seien in erster Linie Halogen, z.B. Chlor oder Brom, oder Sulfonyloxy, z.B. p-Toluolsulfonyloxy, genannt. Funktionell abgewandeltes Carboxy $Y_2$ ist beispielsweise anhydridisiertes Carboxy, wie Halogencarbonyl, Niederalkanoyloxycarbonyl oder Niederalkoxycarbonyloxycarbonyl, Carbamoyl oder Amidino.

Reaktionsfähiges verestertes Hydroxy ist beispielsweise eine vorzugsweise mit starken anorganischen oder organischen Säuren, wie Mineralsäuren, z.B. Halogenwasserstoffsäuren, z.B. Brom- oder Chlorwasserstoffsäure, oder organische Sulfonsäuren, wie gegebenenfalls substituierte Benzolsulfonsäuren, z.B. p-Toluolsulfonsäure, verestertes Hydroxy. Funktionell abgewandelte Carboxyderivate der Formel (IIc) sind beispielsweise entsprechende gegebenenfalls veresterte, amidierte oder anhydridisierte Carboxyverbindungen, wie entsprechende Niederalkoxycarbonyl-, gegebenenfalls substituierte Carbamoyl- oder Halogencarbonyl-, Niederalkanoyloxycarbonyl- oder Niederalkoxycarbonyloxycarbonyl-Derivate.

Als geeignete Kondensationsmittel kommen beispielsweise starke Protonsäuren, wie Mineralsäuren, z.B. Schwefelsäure, Halogenwasserstoffsäuren, Phosphorsäuren, Sulfonsäuren, wie gegebenenfalls substituierte Benzolsulfonsäuren, oder Carbonsäuren, wie Niederalkancarbonsäuren, oder Anhydride von Mineralsäuren, wie entsprechende Anhydride von Phosphorsäuren, Kohlensäure oder Schwefelsäure, z.B. Phosphorpentachlorid, Phosphoroxychlorid, Phosphorpentoxid, Thionylchlorid oder Phosgen, in Betracht.

Die Umsetzung erfolgt in üblicher Weise, erforderlichenfalls unter Erwärmen und in einem inerten Lösungsmittel, wie einem halogenierten Kohlenwasserstoff.

In einer bevorzugten Verfahrensweise gelangt man zu Verbindungen der Formel (II), worin $X_1$ und $X_2$ jeweils Oxy bedeuten, indem man Verbindungen der Formel (IIa), worin $Y_1$ Hydroxy darstellt, mit Säurehalogeniden der Formel (IIc), insbesondere den entsprechenden Säurehalogeniden, umsetzt. Aus den so erhältlichen Verbindungen der Formel (IId) können mit Ammoniak in Gegenwart einer Säure, wie Eisessig, die entsprechenden Verbindungen der Formel (I) gebildet werden.

Insbesondere gelangt man zu Verbindungen der Formel (II), worin $X_1$ Oxy und $X_2$ Imino darstellen, indem man eine Verbindung der Formel (IIa), worin $Y_1$ reaktionsfähiges verestertes Hydroxy, in erster Linie Halogen darstellt, mit einer entsprechender Verbindung der Formel (IIb), worin $Y_2$ Carbamoyl bedeutet, unter den vorstehend angegebenen Reaktionsbedingungen umsetzt.

Insbesondere gelangt man zu Verbindungen der Formel (II), worin $X_1$ Imino und $X_2$ Oxo bedeuten, durch Umsetzung einer Verbindung der Formel (IIa), worin $Y_1$ Amino darstellt, einem Salz und/oder Tautomeren davon mit einer Verbindung der Formel (IIb), worin $Y_2$ anhydridisiertes Carboxy, in erster Linie Halogencarbonyl bedeutet unter den angegebenen Reaktionsbedingungen.

Verbindungen der Formel (II), worin $X_1$ und $X_2$ Imino bedeuten, sind bevorzugt erhältlich, wenn man eine Verbindung der Formel (IIa), worin $Y_1$ gegebenenfalls reaktionsfähiges verestertes Hydroxy, in erster Linie Hydroxy, bedeutet, ein Salz und/oder Tautomeres davon mit einer Verbindung der Formel (IIb), worin $Y_2$ Amidino bedeutet umsetzt.

Die Ausgangsstoffe der Formel (IIa), (IIb) und (IIc) sind bekannt oder können nach an sich bekannten Methoden hergestellt werden.

So lassen sich beispielsweise Verbindungen der Formel (IIa) durch Esterkondensation von veresterten Säuren der Formeln $R_1$-$CH_2$-COOH bzw. $R_2$-$CH_2$-COOH mit veresterten Säuren der Formeln $R_1$-COOH bzw. $R_2$-COOH, vorzugsweise in Gegenwart einer Base erhalten. Das resultierende α-Methylenketon der Formel

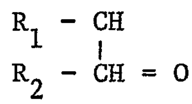

(IIe)

wird beispielsweise bromiert und somit in eine Verbindung der Formel (IIa) bzw. ein Salz, z.B. Hydrohalogenid, davon überführt, worin $Y_1$ Brom bedeutet.

Verbindungen der Formel I oder Salze davon kann man weiterhin herstellen, indem man beispielsweise eine Verbindung der Formel

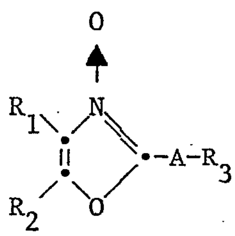

(III)

oder ein Salz davon zu einer Verbindung der Formel I reduziert und gewünschtenfalls die verfahrensgemäss erhältliche freie Verbindung der Formel (I) in eine andere freie Verbindung überführt, eine verfahrensgemäss erhältliche freie Verbindung in ein Salz oder ein verfahrensgemäss erhältliches Salz in die freie Verbindung oder in ein anderes Salz überführt und gewünschtenfalls ein verfahrensgemäss erhältliches Isomerengemisch in seine Komponenten auftrennt.

Die Reduktion erfolgt nach an sich bekannten Verfahren. So behandelt man Verbindungen der Formel (III) oder deren Salze mit Wasserstoff in Gegenwart eines Hydrierungskatalysators oder mit einem Dithionit, z.B. Natriumdithionit, oder mit einem Phosphorhalogenid, z.B. Phosphortrichlorid. Als Hydrierungskatalysatoren lassen sich beispielsweise Elemente der VIII. Nebengruppe sowie Derivate davon, wie Platin, Palladium oder Palladiumchlorid, die gegebenenfalls auf

einem üblichen Trägermaterial, wie Aktivkohle oder Erdalkalimetallverbindungen, z.B. Bariumcarbonat, aufgezogen sind, oder Raney-Nickel, verwenden. Ebenso kann die Reduktion mit einem System aus einem geeigneten unedlen Metall und einer Protonsäure, beispielsweise mit Zink/Eisessig, durchgeführt werden.

Die Reduktion lässt sich erforderlichenfalls unter Kühlen oder Erwärmen, beispielsweise in einem Temperaturbereich von etwa 0° bis etwa 150°C, in einem inerten Lösungsmittel, wie einem halogenierten Kohlenwasserstoff, z.B. Chloroform, Tetrachlorkohlenstoff oder Chlorbenzol, oder einem Ether, wie Dimethoxyethan, Diethylether, Dioxan oder Tetrahydrofuran, und/oder unter Inertgas, z.B. Stickstoff, durchführen.

Die Ausgangsstoffe der Formel (III) oder deren Salze sind in an sich bekannter Weise erhältlich, beispielsweise in dem eine Verbindung der Formel

$$\begin{array}{l} R_1 - C = N - OH \\ \qquad \;| \\ R_2 - C = O \end{array} \qquad (IIIa) \quad ,$$

ein Tautomeres oder ein Salz davon mit einem Aldehyd der Formel $R_3$-A-C(=O)-H (IIIb), gegebenenfalls bei erhöhter Temperatur und in Gegenwart einer Säure, wie Mineralsäure, z.B. Salzsäure, Sulfonsäure, z.B. p-Toluolsulfonsäure, oder Carbonsäure, z.B. Essigsäure, umsetzt.

Verbindungen der Formel I lassen sich ferner herstellen, indem man in einer Verbindung der Formel

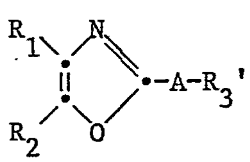 (IV) ,

worin $R_3'$ einen in $R_3$ überführbaren Rest bedeutet, den Rest $R_3'$ in einen Rest $R_3$ überführt und gewünschtenfalls die verfahrensgemäss erhältliche freie Verbindung der Formel (I) in eine andere freie Verbindung überführt, eine verfahrensgemäss erhältliche freie Verbindung in ein Salz oder ein verfahrensgemäss erhältliches Salz in die freie Verbindung oder in ein anderes Salz überführt und gewünschtenfalls ein verfahrensgemäss erhältliches Isomerengemisch in seine Komponenten auftrennt.

Derartige Gruppen $R_3'$ sind beispielsweise durch Solvolyse oder Oxidation in gegebenenfalls verestertes oder amidiertes Carboxy $R_3$ überführbare Gruppen. Als solvolytisch in $R_3$ überführbare Gruppen kommen von $R_3$ verschiedenes funktionell abgewandeltes Carboxy in Frage; zu nennen sind in erster Linie Cyano, anhydridisiertes Carboxy, wie Halogencarbonyl, Niederalkanoyloxycarbonyl oder Niederalkoxycarbonyloxycarbonyl, gegebenenfalls substituiertes Amidino, wie Niederalkylamidino, gegebenenfalls verestertes Thiocarboxy, wie Niederalkylthiocarbonyl, gegebenenfalls verestertes Dithiocarboxy, gegebenenfalls substituiertes Thiocarbamoyl, wie Mono- oder Diniederalkyl-thiocarbamoyl, gegebenenfalls verestertes oder anhydridisiertes Carboximidoyl, wie Niederalkoxy- oder Halogeniminocarbonyl, oder sich von Orthoameisensäureester ableitende Reste, wie Triniederalkoxy- oder Trihalogenmethyl. Solche Gruppen können durch Solvolyse, z.B. Hydrolyse oder Alkoholyse, ferner Ammono- oder Aminolyse, in einen Rest $R_3$ überführt werden.

Solvolysemittel sind beispielsweise Wasser, der gewünschten veresterten Carboxygruppe entsprechende Alkohole, Ammoniak oder der gewünschten amidierten Carboxygruppe entsprechende Amine.

Die Behandlung mit einem entsprechenden Solvolysemittel wird gegebenenfalls in Gegenwart einer Säure oder Base, gegebenenfalls unter Kühlen oder Erwärmen oder erforderlichenfalls in einem inerten Lösungs- oder Verdünnungsmittel durchgeführt. Als Säuren eignen sich beispielsweise anorganische oder organische Protonsäuren, wie Mineralsäuren, z.B. Schwefelsäure oder Halogenwasserstoffsäure, wie Sulfonsäure, z.B. Niederalkan- oder gegebenenfalls substituierte Benzolsulfonsäure, oder wie Carbonsäuren, wie Niederalkancarbonsäuren. Als Basen können

beispielsweise Hydroxide, wie Alkalimetallhydroxide, verwendet werden.

So können beispielsweise die Cyanogruppe, gegebenenfalls verestertes Thiocarboxy, gegebenenfalls verestertes Dithiocarboxy, gegebenenfalls substituiertes Thiocarbamoyl, gegebenenfalls verestertes oder anhydridisiertes Carboximidoyl, oder sich von Orthoameisensäure ableitende Reste $R_3'$, gegebenenfalls in Gegenwart einer Protonsäure zu Carboxy $R_3$ hydrolysiert werden, während beispielsweise Cyano, gegebenenfalls S-verestertes Thiocarboxy oder anhydridisiertes Carboxy $R_3'$ mit einem geeigneten Alkohol, gegebenenfalls in Gegenwart einer Protonsäure, zu verestertem Carboxy $R_3$ alkoholysiert werden können. Amidiertes Carboxy $R_3$ kann z.B. auch durch Aminolyse bzw. durch Behandeln mit einem geeigneten Amin aus Cyano, wobei beispielsweise in Gegenwart einer Lewissäure, wie Aluminiumchlorid, gearbeitet wird, oder anhydridisiertem Carboxy erhalten werden.

Weitere in Carboxy bzw. verestertes Carboxy $R_3$ überführbare Gruppen sind beispielsweise oxidativ in diese überführbare Reste, wie gegebenenfalls hydratisiertes bzw. acetalisiertes Formyl. Diese kann vorteilhaft im Verlaufe der Oxidationsreaktion, z.B. aus der Acylgruppe einer α,β-ungesättigten oder α,β-dihydroxilierten aliphatischen oder araliphatischen Carbonsäure, einer gegebenenfalls an der Hydroxygruppe veresterten Formylgruppe, in situ gebildet oder aus einem ihrer funktionellen Derivate, z.B. einem ihrer Acetale, Acylale oder Imine in Freiheit gesetzt werden. Acylgruppen von α,β-ungesättigten oder α,β-dihydroxylierten Carbonsäuren sind beispielsweise Acylgruppen von α,β-ungesättigten aliphatischen Mono- oder Dicarbonsäuren, z.B. Acryloyl, Crotonyl oder die Acylgruppe der gegebenenfalls funktionell abgewandelten Fumar- oder Maleinsäure, Acylgruppen von α,β-ungesättigten araliphatischen Carbonsäuren, z.B. gegebenenfalls substituiertes Cinnamoyl, oder Acylgruppen von aliphatischen α,β-Dihydroxydicarbonsäuren, wie der Weinsäure, oder monofunktioneller Carboxyderivate, wie Estern oder Amiden, derselben. Veresterte Hydroxymethylgruppen sind beispielsweise an der Hydroxygruppe mit einer Mineralsäure, wie einer Halogenwasserstoffsäure, z.B. mit Chlor- oder Bromwasserstoffsäure, oder mit einer Carbonsäure, z.B. mit Essigsäure

oder der gegebenenfalls substituierten Benzoesäure, veresterte Gruppen. Acetalisierte Formylgruppen sind beispielsweise mit Niederalkanolen oder einem Niederalkandiol acetalisierte Formylgruppen, wie Dimethoxy-, Diethoxy- oder Ethylendioximethyl. Acylalisierte Formylgruppen sind z.B. Diniederalkanoyloxymethyl- ider Dihalogenmethylgruppen, wie Diacetoxymethyl oder Dichlormethyl. Imine von Formylgruppen sind beispielsweise gegebenenfalls substituierte N-Benzylimine oder N-(2-Benzothiazolyl)-imine derselben oder Imine mit 3,4-Di-tert.-butyl-o-chinon. Weitere oxidativ in die Carboxygruppe überführbare Reste sind z.B. gegebenenfalls substituierte, wie in 5-Stellung einer acetalisierten Formylgruppe, wie Diethoxymethyl, aufweisende 2-Furoylgruppen. Zu veresterten Carboxygruppen oxidierbare Gruppen sind veretherte Hydroxymethyl- oder Dihydroxymethylgruppen, wie Niederalkoxymethyl- oder Diniederalkoxymethylgruppen.

Die Oxidation derartiger Gruppen $R_3'$ erfolgt in an sich bekannter Weise, beispielsweise durch Umsetzung in einem geeigneten Oxidationsmittel, beispielsweise in einem inerten Lösungsmittel, wie einer Niederalkancarbonsäure z.B. Essigsäure, einem Keton, z.B. Aceton, einem Ether, z.B. Tetrahydrofuran, einem heterocyclischen Aromaten, z.B. Pyridin, oder Wasser oder einem Gemisch davon, erforderlichenfalls unter Kühlen oder Erwärmen, z.B. von etwa 0° bis etwa 150°C. Als Oxidationsmittel kommen beispielsweise oxidierende Uebergangsmetallverbindungen, insbesondere solche mit Elementen der I., VI., VII., oder VIII. Nebengruppe, in Frage. Als Beispiele seien genannt: Silberverbindungen, wie Silbernitrat, -oxid oder -picolinat, Chromverbindungen, wie Chromtrioxid oder Kaliumdichromat, Manganverbindungen, wie Kaliumferrat, Tetrabutylammonium- oder Benzyl(triethyl)ammoniumpermanganat. Weitere Oxidationsmittel sind beispielsweise geeignete Verbindungen mit Elementen der 4. Hauptgruppe, wie Bleidioxid, oder Halogen-Sauerstoff-Verbindungen, wie Natriumiodat oder Kaliumperiodat.

So führt beispielsweise die Oxidation von Hydroxymethyl bzw. von mit einem Niederalkanol verethertem Hydroxymethyl zu Carboxy bzw. zu mit einem Niederalkanol verestertem Carboxy $R_3$. Diese Umsetzung wird z.B. vorteilhaft mit Kaliumpermanganat in Aceton oder wässrigem Pyridin bei Raumtemperatur durchgeführt. Entsprechend erfolgt die Oxidation von gegebenenfalls hydratisiertem Formyl zu Carboxy $R_3$ z.B. mit Iod in einer methanolischen Kaliumhydroxidlösung. Für die Oxidation von acetalisiertem Formyl zu verestertem Carboxy $R_3$ wird als Oxidationsmittel insbesondere Brom- oder N-Bromsuccinimid verwendet.

Die Ausgangsstoffe der Formel (IV) werden nach an sich bekannten Verfahren hergestellt. Beispielsweise geht man von einem Hydroxyketon der Formel

$$\begin{array}{ccccc} & & H & & \\ & & | & & \\ R_1 & - & C & - & OH \\ & & | & & \\ R_2 & - & C & = & O \end{array} \qquad \text{(IVa)}$$

aus und setzt dieses mit einer Verbindung der Formel $R_3'-A-Y_2'$ (IVb) oder ein Salz davon, worin $Y_2'$ gegebenenfalls funktionell abgewandeltes Carboxy bedeutet, bzw. $R_3'$-A-COOH, einem funktionellen Derivat oder Salz davon und Ammoniak in einem inerten Lösungsmittel und unter Erwärmen in situ zu der Verbindung der Formel (IV) ohne Isolierung von Zwischenprodukten um.

In dem vorstehend beschriebenen Verfahren zur Herstellung von z.B. Verbindungen der Formel IV, kann das Ammoniak, welches überwiegend im Ueberschuss zugegeben wird, auch in Form eines Ammoniak abgebenden Mittels eingesetzt werden, wobei die Freisetzung bei erhöhter Temperatur und gegebenenfalls unter Druck erfolgt. Als Ammoniak abgebende Mittel kommen z.B. Ammoniumsalze von Niederalkancarbonsäuren, vorzugsweise Ammoniumacetat, ferner ein geeignetes Niederalkancarbonsäureamid, insbesondere Formamid, in Frage.

Der Aldehyd der Formel $R_3$-A-C(=O)-H (IIIb) kann in dem vorstehend beschriebenen Herstellungsverfahren für Verbindungen der Formel III beispielsweise auch unter den Reaktionsbedingungen aus einem Oxazinderivat der Formel

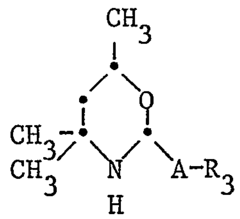

(IIIc)

freigesetzt werden. Die Verbindungen der Formel (IIIc) lassen sich beispielsweise herstellen, indem man 2-Methyl-2,4-pentandiol mit einem Nitril der Formel $R_3$-A-CN in Gegenwart von Schwefelsäure umsetzt. Das dabei gebildete, entsprechend substituierte Dihydro-1,3-oxazin wird in einem Gemisch aus Tetrahydrofuran und Ethanol bei -45°C und einem pH-Wert von etwa 7 unter Einwirkung von Natriumborhydrid zu dem Tetrahydro-1,3-oxazin der Formel (IIIc) reduziert.

Eine erfindungsgemäss erhältliche Verbindung kann in üblicher Weise in eine andere Verbindung der Formel I überführt werden.

So kann man in erfindungsgemäss erhältlichen Verbindungen der Formel I freie und veresterte Carboxygruppen $R_3$ ineinander umwandeln.

Eine freie Carboxylgruppe $R_3$ lässt sich beispielsweise in üblicher Weise z.B. durch Behandeln mit einem Diazoniederalkan, Diniederalkyl-formamidacetal, Alkylhalogenid oder Triniederalkyloxonium-, Triniederalkylcarboxonium- oder Diniederalkylcarboniumsalze, wie Hexachloroantimonat oder Hexafluorophosphat, oder vor allem durch Umsetzung mit dem entsprechenden Alkohol oder einem reaktionsfähigen Derivat, wie einem Carbon-, Phosphorig-, Schweflig- oder Kohlensäureester, z.B. einem Niederalkancarbonsäureester, Triniederalkylphosphit, Diniederalkylsulfit oder dem Pyrocarbonat, oder einem Mineralsäure- oder Sulfonsäureester, z.B. dem Chlor- oder Bromwasserstoffsäure- oder Schwefelsäure-, Benzolsulfonsäure-, Toluolsulfonsäure- oder Methansulfosnäureester, des entsprechenden Alkohols oder einem davon abgeleiteten Olefin, zu einer veresterten Carboxylgruppe $R_3$ verestern.

Die Umsetzung mit dem entsprechenden Alkohol selbst kann vorteilhaft in Gegenwart eines sauren Katalysators erfolgen, wie einer Protonensäure, z.B. von Chlor- oder Bromwasserstoff-, Schwefel-, Phosphor-, Bor-, Benzolsulfon- und/oder Toluolsulfonsäure, oder eine Lewissäure, z.B. von Bortrifluorid-Etherat, in einem inerten Lösungsmittel, insbesondere einem Ueberschuss des eingesetzten Alkohols und erforderlichenfalls in Gegenwart eines wasserbindenden Mittels und/oder unter destillativer, z.B. azeotroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Ueberschuss des eingesetzten Alkoholderivates oder des entsprechenden Alkohols. Ausgehend von einem Mineralsäure- oder Sulfonsäureester setzt man die zu veresternde Säure vorteilhaft in Form eines Salzes, z.B. des Natrium- oder Kaliumsalzes, und arbeitet erforderlichenfalls in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triethylamin oder Pyridin, und/oder in einem inerten Lösungsmittel, wie einer der vorstehend tertiären Stickstoffbasen oder eines polaren Lösungsmittels, z.B. in Dimethylformamid und/oder bei erhöhter Temperatur.

Die Reaktion mit einem Diniederalkyl-formamidacetal, wie Dimethylformamidacetal, erfolgt gegebenenfalls unter Erwärmen, während die Umsetzung mit einem Alkylhalogenid in Gegenwart einer Base, wie einem Amin, z.B. Triethylamin, durchgeführt wird.

Die Umsetzung mit einem Olefin kann beispielsweise in Gegenwart eines sauren Katalysators, z.B. einer Lewissäure, wie Bortrifluorid, einer Sulfonsäure, z.B. von p-Toluolsulfonsäure, oder vor allem eines basischen Katalysators, z.B. von Natrium- oder Kaliumhydroxid, vorteilhaft in einem inerten Lösungsmittel, wie einem Ether, z.B. Diethylether oder Tetrahydrofuran, erfolgen.

Die vorstehend beschriebenen Umwandlungen freier in veresterte Carboxylgruppen $R_2$ können aber auch so durchgeführt werden, dass man eine Verbindung der Formel I, worin $R_3$ Carboxyl ist, zunächst in üblicher Weise in ein reaktionsfähiges Derivat, beispielsweise mittels eines Halogenides des Phosphors oder Schwefels, z.B. mittels Phosportrichlorid oder -bromid, Phosphorpentachlorid oder Thionylchlorid, in ein Säurehalogenid oder durch Umsetzung mit einem entsprechenden Alkohol in einen reaktiven Ester, d.h. Ester mit elektronenanziehenden Strukturen, wie den Ester mit Phenol, Thiophenol, p-Nitrophenol oder Cyanmethylalkohol, überführt und das erhaltene reaktionsfähige Derivat dann in üblicher Weise z.B. wie nachstehend für die Umesterung bzw. gegenseitige Umwandlung veresterter Carboxylgruppen $R_3$ beschrieben, mit einem entsprechenden Alkohol zu der gewünschten Gruppe $R_3$ umsetzt.

Eine veresterte Carboxylgruppe $R_3$ kann in üblicher Weise z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, zur freien Carboxylgruppe $R_3$ überführt werden.

Eine veresterte Carboxylgruppe $R_3$ kann in üblicher Weise, z.B. durch Umsetzung mit einem Metallsalz, wie Natrium- oder Kaliumsalz, eines entsprechenden Alkohols oder mit diesem selbst in Gegenwart eines Katalysators, beispielsweise einer starken Base, z.B. von Natrium- oder Kaliumhydroxid, oder einer starken Säure, wie einer Mineralsäure, z.B. von Salzsäure, Schwefelsäure oder Phosphorsäure, oder einer organischen Sulfonsäure, z.B. von p-Toluolsulfonsäure, oder einer Lewissäure, z.B. von Bortrifluorid-Etherat, zu einer anderen veresterten Carboxylgruppe $R_3$ umgeestert werden.

Weiterhin kann man freies Carboxy bzw. reaktionsfähig funktionelle Carboxyderivate durch Solvolyse mit Ammoniak oder einem primären bzw. sekundären Amin, wobei auch Hydroxylamine bzw. Hydrazine eingesetzt werden können, in üblicher Weise unter Dehydratisierung, gegebenenfalls in Anwesenheit eines Kondensationsmittels, in eine gewünschteamidierte Form überführen. Als Kondensationsmittel werden vorzugsweise Basen verwendet, beispielsweise anorganische Basen, wie Alkalimetallhydroxide, z.B. Natrium- oder Kaliumhydroxid, organische Stickstoffbasen, wie tert.-Amine, z.B. Pyridin, Tributylamin oder N-Dimethylanilin,oder Tetrahalogensilane, wie Tetrachlorsilan.Ebenso kann man in erfindungsgemäss erhältlichen Verbindungen der Formel I, worin $R_3$ amidiertes Carboxy bedeutet, nach an sich bekannten Methoden die Amidbindung spalten und so das Carbamoyl in freies Carboxy überführen. Hierzu arbeitet man in Gegenwart eines Katalysators, beispielsweise einer Base, wie eines Alkalimetall- oder Erdalkalimetallhydroxides oder -carbonates, z.B. Natrium-, Kalium- oder Calciumhydroxid oder -carbonat, oder einer Säure, wie einer Mineralsäure, z.B. Salzsäure, Schwefelsäure oder Phosphorsäure.

Enthält mindestens einer der Substituenten $R_1$, $R_2$ und $R_3$ als zusätzlichen Substituenten Hydroxy, so lässt sich dieser nach an sich bekannter Weise verethern. Die Umsetzung mit einer Alkoholkomponente, z.B. mit einem Niederalkanol, wie Ethanol, in Gegenwart von Säuren,

z.B. Mineralsäure, wie Schwefelsäure, oder von Dehydratisierungsmitteln, wie Dicyclohexylcarbodiimid, führt zu Niederalkoxy.Phenole bzw. deren Salze lassen sich z.B. in Gegenwart von Basen, wie Alkalimetallhydroxiden oder -carbonaten, z.B. Natriumhydroxid oder Kaliumcarbonat, mit Hilfe von Diniederalkylsulfaten, Diazoniederalkanen oder Alkyl- bzw. Arylhalogeniden in entsprechende Niederalkylphenylether bzw. Arylphenylether überführen. Umgekehrt kann man Ether in Alkohole spalten. So entstehen z.B. aus Alkoxyarylverbindungen aromatische Alkohole, indem man die Etherspaltung mittels Säuren, wie Mineralsäuren, z.B. Halogenwasserstoffsäure, wie Bromwasserstoffsäure, oder wie Lewissäuren, z.B. Halogeniden von Elementen der 3. Hauptgruppe, wie Bortribromid, oder mittels Basen, z.B. Niederalkylaminen, wie Methylamin, durchführt.

Weiterhin lässt sich Hydroxy in Niederalkanoyloxy umwandeln, beispielsweise durch Umsetzung mit einer gewünschten Niederalkancarbonsäure, wie Essigsäure oder einem reaktionsfähigen Derivat davon, beispielsweise in Gegenwart einer Säure, wie eine Protonsäure, z.B. Chlor-, Bromwasserstoff-, Schwefel-, Phosphor- oder einer Benzolsulfonsäure, in Gegenwart einer Lewissäure, z.B. von Bortrifluorid-Etherat, oder in Gegenwart eines wasserbindenden Mittels. Umgekehrt kann verestertes Hydroxy, z.B. durch Basen-Katalyse, zu Hydroxy solvolysiert werden.

Erhaltene freie Verbindungen der Formel I können in an sich bekannter Weise in Salze überführt werden. Hydroxy aufweisende Gruppen $R_1$ bzw. $R_2$ sowie Carboxy $R_3$ werden mit entsprechenden Basen, wie Alkalimetallhydroxiden, in die eingangs aufgeführten Salze mit Basen, oder durch Behandeln mit einer wie vorstehend aufgeführten, Säureadditionssalze bildenden Säure in Säureadditionssalze umgewandelt werden.

Erhaltene Salze können in an sich bekannter Weise in die freien Verbindungen umgewandelt werden, z.B. durch Behandeln mit einem sauren Reagenz, wie einer Mineralsäure, bzw. einer Base, z.B. Alkalihydroxid.

Infolge der engen Beziehung zwischen der Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die Verbindung kann, je nach der Wahl der Ausgangsstoffe und Arbeitsweisen, in Form eines der möglichen Isomeren oder als Gemische derselben vorliegen.

Die Verbindung einschliesslich ihrer Salze kann auch in Form ihrer Hydrate erhalten werden oder andere zu Kristallisation verwendete Lösungsmittel einschliessen.

Die Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome als reine optische Isomere, wie Antipoden, oder als Isomerengemische, wie racemate, Diastereoisomerengemische oder Racematgemische, ferner als Tautomere vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegt. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Die Ausgangsstoffe der Formeln II, III und IV, die speziell für die Herstellung der erfindungsgemässen Verbindungen entwickelt wurden, die Verfahren zu ihrer Herstellung sowie ihre Verwendung bilden ebenfalls einen Gegenstand der Erfindung.

Bei den erfindungsgemässen pharmazeutischen Präparaten, welche mindestens einen Wirkstoff oder ein pharmazeutisch verwendbares Salz davon enthalten, handelt es sich vorzugsweise um solche zur topischen Anwendung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applikationsweise ab. Entsprechende Mittel mit einem Konzentrationsbereich von etwa 1 bis etwa 10 % G/G, z.B. in Form von Cremen, Salben oder Lösungen, können beispielsweise 2 bis 3 x täglich appliziert werden.

Als topisch anwendbare pharmazeutische Präparate kommen in erster Linie Cremen, Salben, Pasten, Schäume, Tinkturen und Lösungen in Frage, die von etwa 0,1 bis etwa 10 % des Wirkstoffs enthalten.

Cremen sind Oel-in-Wasser Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline

(Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfessäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyethylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole bzw. Wollwachs, enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoff, z.B. Paraffin, Vaseline und/oder flüssige Paraffine, ferner natürliches oder partialsynthetisches Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cremen und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden z.B. aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glykole und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Aethanol entzogenen Fettsubstanzen, und, falls notwendig, andere Hilft- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die nachfolgenden Beispiele illustrieren die oben beschriebene Erfindung, sie sollen jedoch diese in ihrem Umfang in keiner Weise einschränken. Temperaturen sind in Celsiusgraden angegeben.

Beispiel 1: Eine Lösung von 39,0 g 1-Phenyl-2-(3-pyridyl)-glyoxal (frisch destilliert) in 500 ml Methanol wird unter Rühren bei 0° portionenweise während 5 Stunden mit 2,5 g Natriumborhydrid versetzt. Nach beendeter Zugabe wird die Mischung noch 2 Stunden bei 0° gerührt, 15 Stunden bei 0° stehengelassen und anschliessend mit 200 ml 2-n. Salzsäure versetzt. Man engt die Suspension unter vermindertem Druck zur Trockne ein, versetzt den Rückstand mit Eis und stellt durch Zugabe von 2-n. Natriumcarbonatlösung alkalisch. Die wässrige Suspension wird dreimal mit je 200 ml Ethylacetat extrahiert. Die vereinigten Ethylacetatlösungen werden mit 50 ml Wasser gewaschen, über Magnesiumsulfat getrocknet und unter vermindertem Druck bei 40° eingedampft. Der Rückstand, ein braunes Oel, wird an 500 g Kieselgel chromatographiert. Die Fraktionen 1 und 2, eluiert mit je 1000 ml Toluol-Ethylacetat (1:1), enthalten den Ausgangsstoff (1-Phenyl-2-(3-pyridyl)-glyoxal). Die Fraktionen 3 und 4, eluiert mit je 1000 ml Toluol-Ethylacetat (1:1), werden vereinigt und unter vermindertem Druck zur Trockne eingedampft. Der Rückstand wird aus Ether-Petrolether kristallisiert. Das α-Hydroxy-benzyl-(3-pyridyl)-keton schmilzt bei 109 - 111°. Die Fraktionen 5 und 6, eluiert mit je 1000 ml Toluol-Ethylacetat (1:1), werden verworfen. Die Fraktionen 7 - 14, eluiert mit je 1000 ml Toluol-Ethylacetat (40:60), werden vereinigt und unter vermindertem Druck zur Trockne eingedampft. Den Rückstand kristallisiert man aus Ether-Petrolether. Das α-Hydroxy-phenyl-[(3-pyridyl)-methyl]-keton schmilzt bei 93 bis 95°.

Beispiel 2: Eine Mischung von 6,0 g α-Hydroxyphenyl-[(3-pyridyl)-methyl]-keton und 1 ml Triethylamin in 80 ml wasserfreiem Benzol wird unter Rühren und Einleiten von Stickstoff bei 10° mit einer Lösung von 6,0 g Dimethyl-malonsäure-mono-ethylester-chlorid in 30 ml wasserfreiem Benzol während 20 Minuten versetzt drei Stunden bei Raumtemperatur gerührt, mit 30 ml Ethylacetat und 90 ml Wasser versetzt und nochmals eine Stunde bei Raumtemperatur gerührt. Man trennt die wässrige Phase ab und wäscht sie zweimal mit je 20 ml Ethylacetat. Die vereinigten organischen Phasen werden mit 30 ml gesättigter Kochsalzlösung, 30 ml 2-n. Kaliumbicarbonat und nochmals 30 ml gesättigter Kochsalzlösung gewaschen, über Magnesiumsulfat getrocknet und unter vermindertem Druck eingeengt. Den Rückstand, ein Oel, chromatographiert man an 200 g Kieselgel. Die Fraktionen 1 bis 2, eluiert mit je 200 ml Chloroform, werden verworfen. Die Fraktionen 3 bis 9, eluiert mit Chloroform-Methanol (99:1), werden vereinigt und unter vermindertem Druck zur Trockne eingedampft. Der Rückstand, der Dimethyl-malonsäure-monoethylester-[α-benzoyl-(3-pyridyl-methyl)-ester liegt als Oel vor.

Analog wird erhalten: Methyl-malonsäure-mono-ethylester-[α-benzoyl-(3-pyridyl-methyl)-ester (Oel), ausgehend von α-Hydroxy-phenyl-[(3-pyridyl)-methyl]-keton und Methylmalonsäuremono-ethylester-chlorid.

Malonsäure-mono-ethylester-[α-benzoyl-(3-pyridyl-methyl)-ester (Oel), ausgehend von α-Hydroxy-phenyl-[(3-pyridyl)-methyl]-keton und Malonsäure-mono-ethylester-chlorid.

Beispiel 3: Eine Mischung von 3,1 g Dimethylmalonsäure-mono-ethylester-[α-benzoyl-(3-pyridyl-methyl)-ester, 2,4 g Ammoniumacetat und 24 ml Eisessig wird unter Rühren 2 Stunden bei einer Badtemperatur von 140° gerührt. Man kühlt ab, giesst das Reaktionsgemisch

auf 100 ml Eiswasser und stellt mit konzentrierter wässriger Ammoniaklösung auf pH 8,0. Man extrahiert dreimal mit je 70 ml Ethylacetat und wäscht die Ethylacetatextrakte zweimal mit je 20 ml Wasser. Die vereinigten organischen Phasen werden über Magnesiumsulfat getrocknet und unter vermindertem Druck zur Trockne eingedampft. Den Rückstand chromatographiert man an 100 g Silikagel. Die Fraktionen 1 bis 7, eluiert mit je 100 ml Chloroform, werden verworfen. Die Fraktionen 8 und 9, eluiert mit je 100 ml Chloroform, werden vereinigt und unter vermindertem Druck zur Trockne eingedampft. Der Rückstand, ein Oel, wird mit Petrolether angerieben. Die ausgeschiedenen Kristalle werden abfiltriert und mit kaltem Petrolether nachgewaschen. Der 2-[4-Phenyl-5-(3-pyridyl)-oxazol-2-yl]-2-methyl-propionsäureethylester schmilzt bei 70 bis 71°.

Analog wird erhalten:

2-[4-Phenyl-5-(3-pyridyl)-oxazol-2-yl]-propionsäureethylester (Oel),

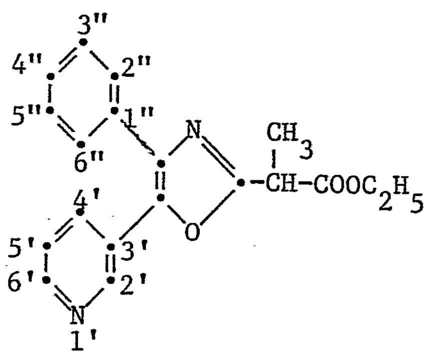

NMR ($CDCl_3$): 8,85 (s, br, H-2'); 8,56 (d, br, H-6'); 7,87 (dt, H-4'); 7,64 (m, H-2",6"); 7,35-7,45 (übrige aromatische H); 7,18 (dd, H-5'); 4,03 (q, $\underline{\underline{CH}}-CH_3$); 1,70 (d, $\underline{\underline{CH_3}}CH$),

ausgehend von Methyl-malonsäure-mono-ethylester-[α-benzoyl-(3-pyridyl-methyl)-ester.

2-[4-Phenyl-5-(3-pyridyl)-oxazol-2-yl]-essigsäureethylester (Oel),

NMR ($CDCl_3$): 8,85 (s, br, H-2'); 8,56 (d, br, H-6'); 7,87 (dt, H-4'); 7,64 (m, H-2",6"); 7,35-7,45 (übrige aromatische H); 7,18 (dd, H-5'); 3,92 (s, $\underline{\underline{CH_2}}CO$),

ausgehend von Malonsäure-mono-ethylester-[α-benzoyl-(3-pyridyl-methyl)-ester.

Beispiel 4: Eine Lösung von 1,6 g 2-[4-Phenyl-5-(3-pyridyl)-oxazol-2-yl]-2-methyl-propionsäureethylester in 20 ml Methylenchlorid wird unter Rühren bei 0° tropfenweise mit einer Lösung von 1,0 g m-Chlorperbenzoesäure in 20 ml Methylenchlorid versetzt. Die Mischung wird zwei Stunden bei 0° gerührt, nochmals mit 0,1 g m-Chlorperbenzoesäure versetzt und 30 Minuten bei 0° gerührt. Die Mischung wird zweimal mit je 10 ml 2-n. Kaliumbicarbonatlösung gewaschen, über Magnesiumsulfat getrocknet und unter vermindertem Druck zur Trockne eingedampft. Den Rückstand, ein Oel, chromatographiert man an 40 g Kieselgel. Die Fraktionen 1 bis 3, eluiert mit je 50 ml Chloroform, werden verworfen. Die Fraktionen 4 bis 11, eluiert mit je 50 ml Chloroform, werden vereinigt und unter vermindertem Druck zur Trockne eingedampft. Der Rückstand, der 2-[4-Phenyl-5-(1-oxido-3-pyridyl)-oxazol-2-yl]-2-methyl-propionsäureethylester liegt als Oel vor.

NMR ($CDCl_3$): 8,39 (t, H-2'); 8,03 (dt, H-6'); 7.10 (dd, H-5'); 7,2-7,6 (m, übrige atomatische H); 1,65 (s, gem. $CH_3$).

Analog wird erhalten:

2-[4-Phenyl-5-(1-oxido-3-pyridyl)-oxazol-2-yl]-propionsäureethylester (Oel), ausgehend von 2-[4-Phenyl-5-(3-pyridyl)-oxazol-2-yl]-propionsäureethylester.

2-[4-Phenyl-5-(1-oxido-3-pyridyl)-oxazol-2-yl]-essigsäureethylester (Oel), ausgehend von 2-[4-Phenyl-5-(3-pyridyl)-oxazol-2-yl]-essigsäure-ethylester.

Beispiel 5: Eine Lösung von 2,1 g 2-[4-Phenyl-5-(1-oxido-3-pyridyl)-oxazol-2-yl]-2-methyl-propionsäureethylester in 10 ml Methanol wird unter Rühren mit 7,2 ml 1-n. Natronlauge versetzt. Die Lösung wird 5 Stunden bei Raumtemperatur gerührt und unter vermindertem Druck zur Trockne eingedampft. Den Rückstand löst man in 50 ml Wasser und extrahiert die wässrige Lösung mit 20 ml Methylenchlorid. Dann wird die wässrige Phase abgetrennt und mit 2-n. Salzsäure genau auf pH 4,0 gestellt. Die Suspension wird 5 Minuten bei 0° gerührt und abfiltriert. Die 2-[4-Phenyl-5-(1-oxido-3-pyridyl)-oxazol-2-yl]-2-methyl-propionsäure schmilzt bei 136 - 137°.

Analog wird erhalten:

2-[4-Phenyl-5-(1-oxido-3-pyridyl)-oxazol-2-yl]-propionsäure aus 2-[4-Phenyl-5-(1-oxido-3-pyridyl)-oxazol-2-yl]-propionsäureethylester.

2-[4-Phenyl-5-(1-oxido-3-pyridyl)-oxazol-2-yl]-essigsäure ausgehend von 2-[4-Phenyl-5-(1-oxido-3-pyridyl)-oxazol-2-yl]-essigsäureethylester.

2-[4-Phenyl-5-(3-pyridyl)-oxazol-2-yl]-2-methyl-propionsäure, Smp. 111-112° (aus Methanol), ausgehend von 2-[4-Phenyl-5-(3-pyridyl)-oxazol-2-yl]-2-methylpropionsäureethylester.

Beispiel 6: In eine Suspension von 11,3 g α-Oximobenzyl-(3-pyridyl)-keton und 12,2 g 2-(2,2-Dimethyl-carbethoxymethyl)-4,4,6-trimethyl-2,3,5,6-tetrahydro-1,3-oxim in 300 ml Eisessig wird ohne Kühlung und unter gutem Rühren Salzsäuregas eingeleitet. Die Temperatur steigt dabei auf 45° und es entsteht eine klare Lösung. In die Lösung wird bei 115° während 30 Minuten Salzsäuregas eingeleitet, dann wird die Lösung 15 Stunden bei 115° erhitzt, abgekühlt und unter verminderten Druck bei 50° zur Trockene eingedampft. Den Rückstand löst man in 300 ml Wasser. Die wässrige Lösung wird mit konzentrierter wässriger Ammoniaklösung alkalisch gestellt und die ausgeschiedenen Kristalle mit 300 ml Ethylacetat-methylenchlorid (1:1) gelöst. Die organische Phase wird mit 50 ml Wasser gewaschen, über Magnesiumsulfat getrocknet und unter vermindertem Druck zur Trockene eingedampft. Den Rückstand kristallisiert man aus Methanol-Ether. Der 2-[4-Phenyl-5-(3-pyridyl)-(3-oxido-oxazol)-2-yl]-2-methyl-propionsäureethylester schmilzt bei 195-210°.

Analog wird erhalten:
2-[4-Phenyl-5-(3-pyrido)-(3-oxido-oxazol)-2-yl]-propionsäureethylester, ausgehend von α-Oximo-benzyl-(3-pyridyl)-keton und 2-(2-Methyl-carbethoxymethyl)-4,4,6-trimethyl-2,3,5,6-tetrahydro-1,3-oxazin,
2-[4-Phenyl-5-(3-pyrido)-(3-oxido-oxazol)-2-yl]-essigsäureethylester, ausgehend von α-Oximo-benzyl-(3-pyridyl)-keton und 2-(carbethoxymethyl)-4,4,6-trimethyl-2,3,5,6-tetrahydro-1,3-oxazin.

Beispiel 7: Eine Lösung von 1,2 g 2-[4-Phenyl-5-(3-pyrido)-(3-oxido-oxazol)-2-yl]-2-methyl-propionsäureethylester in 10 ml Eisessig wird bei 10° unter Rühren portionenweise mit 1,2 g Zinkstaub versetzt. Nach beendeter Zugabe werden noch 0,05 ml konzentrierte Salzsäure zugesetzt. Man erhitzt die Mischung eine Stunde bei 90°, kühlt

ab, filtriert und wäscht mit 5 ml Eisessig nach. Das Filtrat wird unter vermindertem Druck bei 50° zur Trockene eingedampft. Den Rückstand löst man in 20 ml Wasser. Die wässrige Lösung wird mit wässriger konzentrierter Ammoniaklösung alkalisch gestellt. Die Suspension wird dreimal mit je 30 ml Ethylacetat extrahiert. Man wäscht die vereinigten organischen Phasen mit 10 ml Wasser, trocknet sie über Magnesiumsulfat und engt sie unter vermindertem Druck zur Trockene ein. Den Rückstand chromatographiert man an 80 g Silicagel. Die Fraktionen 1-5, eluiert mit je 50 ml Chloroform, werden verworfen. Die Fraktionen 6-9, eluiert mit je 50 ml Chloroform, werden vereinigt und unter vermindertem Druck zur Trockene eingedampft. Der Rückstand kristallisiert nach Anreiben mit Petrolether. Der 2-[4-Phenyl-5-(3-pyridyl)-oxazol-2-yl]-2-methyl-propionsäureethylester schmilzt bei 70-71°.

Beispiel 8: Eine Mischung von 3,6 g $\alpha$-Brom-benzyl-(3-pyridyl)-keton, 1,7 g Dimethylmalonsäure-mono-ethylester-mono-amid und 1,3 g Collidin in 100 ml Xylol wird 15 Stunden bei 120-130° erhitzt, wobei das gebildete Wasser mit einem Wasserabscheider abgetrennt wird. Anschliessend wird das Reaktionsgemisch unter vermindertem Druck zur Trockene eingedampft. Den Rückstand versetzt man mit 30 ml Wasser und konzentrierter wässriger Ammoniaklösung und extrahiert dreimal mit je 40 ml Ethylacetat. Die vereinigten Ethylacetatlösungen werden mit 20 ml Wasser gewaschen, über Magnesiumsulfat getrocknet und unter vermindertem Druck zur Trockene eingedampft. Den Rückstand chromatographiert man an 80 g Silicagel. Die Fraktionen 1-3, euliert mit je 100 ml Chloroform, werden verworfen. Die Fraktionen 4-6, eluiert mit je 100 ml Chloroform, enthalten den 2-[4-Phenyl-5-(3-pyridyl)-oxazol-2-yl]-2-methyl-propionsäureethylester. Sie werden vereinigt und unter vermindertem Druck zur Trockene eingedampft. Den Rückstand verreibt man mit Petrolether und filtriert die ausgeschiedenen Kristalle ab. Smp. 70-71°.

Beispiel 9: Eine Mischung von 3,56 g α-Brom-benzyl-(3-pyridyl)-keton-hydrobromid und 3,66 g Dimethyl-malonsäure-mono-ethylester-Natriumsalz in 100 ml wässerigem Ethanol wird unter Rühren zum Rückfluss erhitzt. Nach Zusatz von 1 ml konzentrierter Schwefelsäure wird die Lösung eine Stunde unter Rückfluss und 15 Stunden bei 50° gerührt. Man kühlt ab und engt unter vermindertem Druck zur Trockne ein. Den Rückstand, ein Oel, chromatographiert man an 250 g Silicagel. Die Fraktionen 1 bis 5, eluiert mit je 300 ml Chloroform, werden verworfen. Die Fraktionen 6 bis 8, eluiert mit je 300 ml Chloroform, werden vereinigt und unter vermindertem Druck zur Trockne eingedampft. Der Rückstand, der Dimethyl-malonsäure-mono-ethylester-[α-nicotinoyl-benzyl]-ester liegt als Oel vor.

Analog wird erhalten:

Methyl-malonsäure-mono-ethylester-[α-nicotinoyl-benzyl]-ester (Oel), ausgehend von α-Brom-benzyl-(3-pyridyl)-keton-hydrobromid und Methyl-malonsäure-mono-ethylester-Natriumsalz.

Malonsäure-mono-ethylester-[α-nicotinoyl-benzyl]-ester (Oel), ausgehend von α-Brom-benzyl-(3-pyridyl)-ketonhydrobromid und Malonsäure-mono-ethylester-Natriumsalz.

Beispiel 10: Eine Mischung von 6,5 g Dimethyl-malonsäure-mono-ethylester-[α-nicotinoyl-benzyl]-ester, 4,8 g Ammoniumacetat und 50 ml Eisessig wird unter Rühren 2 Stunden bei einer Badtemperatur von 140° gerührt. Man kühlt ab, giesst das Reaktionsgemisch auf 400 ml Eiswasser und stellt mit konzentrierter wässriger Ammoniaklösung auf pH 8,0. Man extrahiert dreimal mit je 100 ml Ethylacetat und wäscht die Ethylacetatextrakte zweimal mit je 30 ml gesättigter Kochsalzlösung. Die vereinigten organischen Phasen werden über Magnesiumsulfat getrocknet und unter vermindertem Druck zur Trockene eingedampft. Den Rückstand chromatographiert man an 300 g Silicagel. Fraktion 1, eluiert mit 300 ml Chloroform, wird verworfen. Die Fraktionen 2-4, eluiert mit je 300 ml Chloroform, werden vereinigt

und unter vermindertem Druck zur Trockene eingedampft. Der Rückstand wird mit Petrolether angerieben. Die ausgeschiedenen Kristalle werden abfiltriert und mit kaltem Petrolether nachgewaschen. Der 2-[4-Phenyl-5-(3-pyridyl)-oxazol-2-yl]-2-methyl-propionsäureethylester schmilzt bei 70-71°.

Die Fraktionen 5-10, eluiert mit je 300 ml Chloroform, werden verworfen. Die Fraktionen 11-15, eluiert mit je 300 ml Chloroform, werden vereinigt und unter vermindertem Druck zur Trockene eingedampft. Der Rückstand, der 2-[4-(3-Pyridyl)-5-phenyl-oxazol-2-yl]-2-methyl-propionsäureethylester, kristallisiert beim Anreiben mit kaltem Petrolester. Smp. 41-42°.

Analog wird erhalten:

2-[4-Phenyl-5-(3-pyridyl)-oxazol-2-yl]-propionsäureethylester (Oel) und 2-[4-(3-Pyridyl)-5-phenyl-oxazol-2-yl]-propionsäureethylester (Oel), ausgehend von Methyl-malonsäure-mono-ethylester-[α-nicotinoyl-benzyl]-ester.

2-[4-Phenyl-5-(3-pyridyl)-oxazol-2-yl]-essigsäureethylester (Oel) und 2-[4-(3-Pyridyl-5-phenyl-oxazol-2-yl]-essigsäureethylester (Oel), ausgehend von Malonsäure-mono-ethylester-[α-nicotinoyl-benzyl)-ester.

2-[4-Phenyl-5-(3-pyridyl)-oxazol-2-yl]-2-methyl-propionsäure-tert.-butylester, Oel,
NMR ($CDCl_3$): 8,85 (s, br, H-2'); 8,56 (d, br, H-6'); 7,87 (dt, H-4'); 7,64 (m, H-2",6"); 7,35-7,45 (ürbige aromatische H); 7,18 (dd, H-5'); 1,75 (s, gem. $CH_3$), und

2-[4-(3-Pyridyl)-5-phenyl-oxazol-2-yl]-2-methyl-propionsäure-tert.-butylester, Oel,
NMR ($CDCl_3$) 8,90 (s, br, H-2'); 8,57 (d, br, H-6'); 8,00 (dt, H-4'); 7,55 (m, H-2",6"); 7,35-7,45 (m, übrige aromatische H); 7,32 (dd, H-5'); 1,73 (s, gem. $CH_3$),
ausgehend von Malonsäure-mono-tert.-butylester-[α-nicotinoyl-benzyl]-ester.

Beispiel 11: Analog wie in Beispiel 4 beschrieben wird erhalten:

2-[4-(1-Oxido-3-pyridyl)-5-phenyl-oxazol-2-yl]-2-methyl-propionsäureethylester (Oel), ausgehend von 2-[4-(3-Pyridyl)-5-phenyl-oxazol-2-yl]-2-methyl-propionsäureethylester. Smp. 85-90° (Ether-Petrolether).

2-[4-(1-Oxido-3-pyridyl)-5-phenyl-oxazol-2-yl]-propionsäureethylester, ausgehend von 2-[4-(3-Pyridyl)-5-phenyl-oxazol-2-yl]-propionsäureethylester.

2-[4-(1-Oxido-3-pyridyl)-5-phenyl-oxazol-2-yl]-essigsäureethylester, ausgehend von 2-[4-(3-pyridyl)-5-phenyl-oxazol-2-yl]-essigsäureethylester.

2-[4-Phenyl-5-(1-oxido-3-pyridyl)-oxazol-2-yl]-2-methylpropionsäure-tert.-butylester, Oel, NMR ($CDCl_3$): 8,39 (t, H-2'); 8,03 (dt, H-6'); 7,10 (dd, H-5'); 7,2-7,6 (m, übrige aromatische H); 1,65 (s, gem. $CH_3$); ausgehend von
2-(4-Phenyl-5-(3-pyridyl)-oxazol-2-yl]-2-methyl-propionsäure-tert.-butylester,
2-[4-(1-Oxido-3-pyridyl)-oxazol-2-yl]-2-methyl-propionsäure-tert.-butyl-ester, Oel, ausgehend von
2-[4-(3-Pyridyl)-5-phenyl-oxazol-2-yl]-2-methyl-propionsäure-tert.-butylester.

Beispiel 12: Eine Lösung von 6,2 g α-Aminobenzyl-(3-pyridyl)-keton in 100 ml wasserfreiem Tetrahydrofuran wird unter Rühren und Einleiten von Stickstoffgas mit 8,8 g Dimethyl-malonsäure-monoethylester-chlorid und 7,2 ml N,N-Diisopropyl-ethylamin versetzt. Die Mischung wird 15 Stunden gerührt und unter vermindertem Druck zur Trockene eingedampft. Den Rückstand chromatographiert man an 500 g Silicagel. Die Fraktionen 1-6, eluiert mit je 400 ml Chloroform, werden verworfen. Die Fraktionen 7-10, eluiert mit je 400 ml Chloroform, werden vereinigt und unter vermindertem Druck zur Trockene eingedampft. Das N-[α-(3-Pyridyl)-phenacyl]-dimethyl-malonsäure-mono-ethylester-mono-amid, Oel.

Analog wird erhalten:

N-[α-(3-Pyridyl)-phenacyl]-methylmalonsäure-mono-ethylester-mono-amid (Oel), ausgehend von α-Amino-benzyl-(3-pyridyl)-keton und Methylmalonsäure-mono-ethylester-chlorid.

N-[α-(3-Pyridyl)-phenacyl]-malonsäure-mono-ethylester-mono-amid, ausgehend von α-Amino-benzyl-(3-pyridyl)-keton und Malonsäure-mono-ethylester-chlorid.

Beispiel 13: Eine Lösung von 2,1 g N-[α-(3-Pyridyl)-phenacyl]-dimethyl-malonsäure-mono-ethylester-mono-amid in 40 ml Phosphoroxychlorid wird 4 Stunden unter Rückfluss erhitzt. Man kühlt ab und giesst die Mischung auf Eis. Das ausgeschiedene Oel wird mit 100 ml Ethylacetat extrahiert. Die Ethylacetatlösung wird zweimal mit je 20 ml Wasser, zweimal mit je 20 ml 1-n. Natriumkarbonatlösung und nochmals mit 20 ml Wasser extrahiert, über Magnesiumsulfat getrocknet und unter vermindertem Druck eingedampft. Den Rückstand chromatographiert man an 70 g Silicagel. Die Fraktionen 1-5, eluiert mit je 80 ml Chloroform, werden verworfen. Die Fraktionen 6-8, eluiert mit je 80 ml Chloroform, werden vereinigt und unter vermindertem Druck zur Trockene eingedampft. Den Rückstand verreibt man mit Petrolether, wobei der 2-[4-Phenyl]5-(3-pyridyl)-oxazol-2-yl]-2-methyl-propionsäureethylester kristallisiert. Smp. 70-71°.

Analog wird hergestellt:
2-[4-Phenyl-5-(3-pyridyl)-oxazol-2-yl]-propionsäureethylester (Oel), ausgehend von N-[α-(3-Pyridyl)-phenacyl]-methyl-malonsäure-mono-ethylester-mono-amid.

2-[4-Phenyl-5-(3-pyridyl)-oxazol-2-yl]-essigsäureethylester (Oel), ausgehend von N-[α-(3-Pyridyl)-phenacyl]-malonsäure-mono-ethylester-mono-amid.

α-Amino-benzyl-(3-pyridyl)-keton kann auf folgende Weise hergestellt werden:

10,8 g Benzyl-(3-pyridyl)-keton werden zusammen mit 40 ml Pyridin und einer Lösung von 8 g Hydroxylaminhydrochlorid in 15 ml Pyridin 6 Stunden bei 100° gerührt. Das Reaktionsgemisch wird auf Eis/Wasser gegossen und 15 Minuten nachgerührt. Die ausgefallenen Kristalle werden abgenutscht, mit Wasser gewaschen und im Hochvakuum getrocknet. Man erhält das Benzyl-(3-pyridyl)-keton-oxim vom Smp. 122-126°.

Zu einer bei -10° gerührten Lösung von 8,5 g Benzyl-(3-pyridyl)-keton-oxim in 20 ml Pyridin wird innerhalb von 5 Minuten eine Lösung von 7,7 g p-Toluolsulfochlorid in 15 ml Pyridin zugetropft. Das Reaktionsgemisch wird 24 Stunden im Eisschrank aufbewahrt und dann auf Eis/Wasser gegossen. Nach einigem Rühren und Verreiben erstarrt das ausgefallene Oel zu Kristallen. Diese werden abgenutscht, mit Wasser gewaschen und im Hochvakuum getrocknet. Man erhält den Benzyl-(3-pyridyl)-keton-oxim-p-toluolsulfonsäureester, der ohne weitere Reinigung in der nächsten Stufe eingesetzt wird.

11,6 g roher Benzyl-(3-pyridyl)-keton-oxim-p-toluolsulfoester werden in 90 ml absolutem Ethanol suspendiert. Dann werden bei 0° unter Rühren die Lösung von 3,7 g Kalium-tert.-butylat in 30 ml absolutem Ethanol zugetropft. Das Reaktionsgemisch wird 2 Stunden bei 0° gerührt. Die Suspension wird abgenutscht und das Filtrat, welches das gewünschte α-Amino-benzyl-(3-pyridyl)-keton enthält, unter vermindertem Druck zur Trockene eingedampft.

Beispiel 14: Eine Salbe, enthaltend 5 % 2-[4-Phenyl-5-(1-oxido-3-pyridyl)-oxazol-2-yl]-2-methyl-propionsäureethylester, kann wie folgt hergestellt werden:

Zusammensetzung:

| | |
|---|---|
| Wirkstoff | 5,0 % |
| Vaseline | 45,0 % |
| Paraffinöl | 19,6 % |
| Cetylalkohol | 5,0 % |
| Bienenwachs | 5,0 % |
| Sorbitan-sesquioleat | 5,0 % |
| p-Hydroxybenzoesäureester | 0,2 % |
| Wasser , entmineralisiert bis zu | 100,0 % |

Die Fettstoffe und Emulgatoren werden zusammengeschmolzen. Das Konservierungsmittel wird in Wasser gelöst, und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

Beispiel 15: Eine Crème, enthaltend 10 % 2-[4-Phenyl-5-(3-pyridyl)-oxazol-2-yl]-2-methyl-propionsäureethylester, kann wie folgt hergestellt werden:

Zusammensetzung:

| | |
|---|---|
| Wirkstoff | 10,0 % |
| Isopropylpalmitat | 8,0 % |
| Cetylpalmitat | 1,5 % |
| Siliconöl 100 | 0,5 % |
| Sorbitanmonostearat | 3,0 % |
| Polysorbat 60 | 3,5 % |
| 1,2-Propylenglykol PH | 20,0 % |
| Acrylsäurepolymerisat | 0,5 % |
| Triethanolamin | 0,7 % |
| Wasser, entmineralisiert bis zu | 100,0 % |

Das Acrylsäurepolymerisat wird in einem Gemisch aus entmineralisiertem Wasser und 1,2-Propylenglykol suspendiert. Unter Rühren wird hierauf Triethanolamin zugegeben. wodurch ein Schleim erhalten wird. Ein Gemisch aus Isopropylpalmitat, Cetylpalmitat, Siliconöl, Sorbitanmonostearat und Polysorbat wird auf ca. 75° erwärmt und unter Rühren in den gleichfalls auf ca. 75° erwärmten Schleim eingearbeitet. Die auf Raumtemperatur abgekühlten Crème-Grundlage wird hierauf zur Herstellung eines Konzentrates mit dem Wirkstoff verwendet. Das Konzentrat wird mittels eines Durchlaufhomogenisators homogenisiert und dann protionsweise der Grundlage hinzugefügt.

Beispiel 16: Eine Crème, enthaltend 5 % 2-[4-Phenyl-5-(1-oxido-3-pyridyl)-oxazol-2-yl]-2-methyl-propionsäureethylester, kann wie folgt erhalten werden.

Zusammensetzung:

| | |
|---|---|
| Wirkstoff | 5,0 % |
| Cetylpalmitat PH | 2,0 % |
| Cetylalkohol PH | 2,0 % |
| Triglyceridgemisch gesättigter mittelfettiger Fettsäuren | 5,0 % |
| Stearinsäure | 3,0 % |
| Glycerinstearat PH | 4,0 % |
| Cetomacrogol 1000 | 1,0 % |
| mikrokristalline Cellulose | 0,5 % |
| 1,2-Propylenglykol, dest. | 20,0 % |
| Wasser, entmineralisiert, bis zu | 100,0 % |

Cetylalkohol, Cetylpalmitat, das Triglyceridgemisch, Stearinsäure und Glycerinstearat werden zusammengeschmolzen. Die mikrokristalline Cellulose wird in einen Teil des Wassers dispergiert. Im restlichen Teil des Wassers wird Cetomacrogol gelöst und das Propylenglykol sowie der Schleim dazu gemischt. Die Fettphase wird anschliessend unter Rühren zu Wasserphase gegeben und kaltgerührt. Schliesslich wird der Wirkstoff mit einem Teil der Grundlage angerieben und hierauf die Anreibung in den Rest der Crème eingearbeitet.

Beispiel 17: Ein transparentes Hydrogel enthaltend 5 % 2-[4-Phenyl-5-(1-oxido-3-pyridyl)-oxazol-2-yl]-2-methyl-propionsäureethylester wird wie folgt hergestellt:

Zusammensetzung:

| | |
|---|---|
| Wirkstoff | 5 % |
| Propylenglykol | 10 - 20 % |
| Isopropanol | 20 % |
| Hydroxypropyl-methylcellulose | 2 % |
| Wasser | ad 100 % |

Die Hydroxypropyl-methylcellulose wird im Wasser gequollen. Der Wirkstoff wird in einem Gemisch aus Isopropanol und Propylenglykol gelöst. Anschliessend wird die Wirkstofflösung mit einem gequollenen Cellulose-Derivat vermischt und, wenn erwünscht, mit Riechstoffen (0,1 %) versetzt.

Beispiel 18: Ein transparentes Hydrogel, enthaltend 5 % 2-[4-Phenyl-5-(1-oxido-3-pyridyl)-oxazol-2-yl]-2-methyl-propionsäureethylester, wird wie folgt hergestellt:

Zusammensetzung:

| | |
|---|---|
| Wirkstoff | 5 % |
| Propylenglykol | 20 % |
| Isopropanol | 20 % |
| Acrylsäurepolymerisat | 2 % |
| Triethanolamin | 3 % |
| Wasser | ad 100 % |

Acrylsäurepolymerisat und Wasser werden dispergiert und mit Triethanolamin neutralisiert. Der Wirkstoff wird in einem Gemisch aus Isopropanol und Propylenglykol gelöst. Anschliessend wird die Wirkstofflösung mit dem Gel vermischt, wobei, wenn erwünscht, Riechstoff (0,1 %) zugegeben werden kann.

Beispiel 19: Ein Schaumspray, enthaltend 1 % 2-[4-Phenyl-5-(1-oxido-3-pyridyl)-oxazol-2-yl]-2-methyl-propionsäure, kann folgendermassen hergestellt werden:

Zusammensetzung:

| | |
|---|---|
| Wirkstoff | 1,00 % |
| Cetylalkohol PH | 1,70 % |
| Paraffinöl, dickflüssig | 1,00 % |
| Isopropylmyristat | 2,00 % |
| Cetomacrogol 1000 | 2,40 % |
| Sorbitanmonostearat | 1,50 % |
| 1,2-Propylenglykol PH | 5,00 % |
| Methylparaben | 0,18 % |
| Propylparaben | 0,02 % |
| Chemoderm 314 | 0,10 % |
| Wasser, entmineralisiert, bis zu | 100,00 % |

Cetylalkohol, Paraffinöl, Isopropylmyristat, Cetomacrogol und Sorbitanstearat werden zusammengeschmolzen. Methyl- und Propylparaben werden in heissem Wasser gelöst. Die Schmelze und die Lösung werden anschliessend vermischt. Der Wirkstoff, in Propylenglykol suspendiert, wird in die Grundlage eingearbeitet. Anschliessend wird Chemoderm zugeführt und mit Wasser auf das Endgewicht ergänzt.

Abfüllung:

20 ml des Gemisches wird in eine Aluminiumblockdose eingefüllt. Die Dose wird mit einem Ventil versehen, und das Treibgas wird unter Druck eingefüllt.

Beispiel 20: Eine Mischung von 4,0 g α-Hydroxyphenyl-[(3-pyridyl)-methyl]-keton und 0,6 ml Triethylamin in 70 ml wasserfreiem Benzol wird unter Rühren und Einleiten von Stickstoff bei 10° mit einer Lösung von 3,7 g 2-Ethoxymethyl-2-methyl-propionsäurechlorid in 20 ml wasserfreiem Benzol während 20 Minuten versetzt. Anschliessend wird die Mischung während drei Stunden bei Raumtemperatur gerührt, mit 30 ml Ethylacetat und 60 ml Wasser versetzt und nochmals eine Stunde bei Raumtemperatur gerührt. Man trennt die wässrige Phase ab und wäscht sie zweimal mit je 20 ml Ethylacetat. Die vereinigten organischen Phasen werden mit 20 ml gesättigter Kochsalzlösung, 20 ml 2-n. Kaliumbicarbonatlösung und nochmals 20 ml gesättigter Kochsalzlösung gewaschen, über Magnesiumsulfat getrocknet und unter vermindertem Druck eingeengt. Den Rückstand (2-Ethoxymethyl-2-methyl-propionsäure-[α-benzoyl-(3-pyridyl-methyl)]-ester, (Oel), löst man in 40 ml Eisessig, setzt 4,1 g Ammoniumacetat zu und erhitzt die Mischung unter Rühren 2 Stunden bei einer Badtemperatur von 140°. Man kühlt ab, giesst das Reaktionsgemisch auf 150 ml Eiswasser und stellt mit konzentrierter wässriger Ammoniaklösung auf pH 8,0. Man extrahiert dreimal mit je 100 ml Ethylacetat und wäscht die Ethylacetatextrakte zweimal mit je 20 ml Wasser. Die vereinigten organischen Phasen werden über Magnesiumsulfat getrocknet und unter vermindertem Druck zur Trockne eingedampft. Den Rückstand chromatographiert man an 150 g Silicagel. Die Fraktionen 1-5, eluiert mit je 150 ml Chloroform, werden verworfen. Die Fraktionen 6-9, eluiert mit je 150 ml Chloroform, werden vereinigt und unter vermindertem Druck zur Trockne eingedampft. Der Rückstand, das 2-[4-(Phenyl-5-(3-pyridyl)-oxazol-2-yl]-2-methyl-1-ethoxy-propan, liegt als Oel vor.

Beispiel 21: Eine Lösung von 2,0 g (2-[4-Phenyl-5-(3-pyridyl)-oxazol-2-yl]-2-methyl-1-ethoxy-propan in 40 ml Aceton und 12 ml Wasser wird bei Raumtemperatur unter kräftigem Rühren portionenweise mit Kaliumpermanganat versetzt, bis keine Entfärbung mehr zu beobachten ist.

Anschliessend wird die Mischung 10 Stunden bei Raumtemperatur gerührt und danach abfiltriert. Man engt das Filtrat unter 11 Torr bei 50° zur Trockne ein. Den Rückstand versetzt man mit 15 ml Eiswasser und extrahiert mit Ethylacetat. Die organische Phase wird mit gesättigter Natriumchlorid-Lösung gewaschen, über Natriumsulfat getrocknet und unter vermindertem Druck zur Trockne eingedampft. Den Rückstand chromatographiert man an 70 g Silicagel. Die Fraktionen 1-3, eluiert mit je 50 ml Chloroform, werden verworfen. Die Fraktionen 4-8, eluiert mit je 50 ml Chloroform, werden vereinigt und unter vermindertem Druck zur Trockne eingedampft. Den Rückstand verreibt man mit kaltem Petrolether. Nach Stehenlassen über Nacht werden die ausgeschiedenen Kristalle abfiltriert. Der 2-[4-Phenyl-5-(3-pyridyl)-oxazol-2-yl]-2-methyl-propionsäureethylester schmilzt bei 70-71°.

Beispiel 22: Eine Mischung von 1,0 g 2-[4-Phenyl-5-(3-pyridyl)-oxazol-2-yl]-2-methylpropionsäure, 0,6 g Triethylenglykol-monomethylether und 40 mg 4-Dimethylaminopyridin in 30 ml Methylenchlorid wird bei Raumtemperatur unter gutem Rühren und Einleiten von Stickstoff mit 0,7 g Dicyclohexylcarbodiimid versetzt. Man rührt 48 Stunden bei Raumtemperatur, setzt 40 ml Toluol zu und filtriert ab. Das Filtrat wird unter vermindertem Druck zur Trockne eingedampft. Den Rückstand löst man in 20 ml Methylenchlorid. Die Methylenchloridlösung wird mit 5 ml 0,1-n Natriumcarbonatlösung und 5 ml Wasser gewaschen, über Magnesiumsulfat getrocknet und unter vermindertem Druck zur Trockne eingedampft. Der Rückstand, der 2-[4-Phenyl-5-(3-pyridyl)-oxazol-2-yl]-2-methyl-propionsäure-2-[2-(2-methoxyethoxy)-ethoxy]-ethylester liegt als Oel vor,
NMR ($CDCl_3$): 8,85 (s, br, H-2'); 8,56 (d, br, H-6'); 7,87 (dt, H-4'); 7,64 (m, H-2",6"); 7,35-7,45 (übrige aromatische H); 7,18 (dd, H-5'); 1,75 s, gem.$CH_3$).

Analog wird erhalten:

2-[4-Phenyl-5-(1-oxido-3-pyridyl)-oxazol-2-yl]-2-methyl-propionsäure-2-[2-(2-methoxyethoxy)-ethoxy]-ethylester (Oel),
NMR ($CDCl_3$): 8,39 (t, H-2'); 8,03 (dt, H-6'); 7,10 (dd, H-5');, 7,2-7,6 (m, übrige aromatische H); 1,65 (s, gem. $CH_3$), und

2-[4-Phenyl-5-(1-oxido-3-pyridyl)-oxazol-2-yl]-2-methyl-propionsäure-2-[2-(2-hydroxyethoxy)-ethoxy]-ethylester, (Oel),

NMR ($CDCl_3$): 8,39 (t, H-2'); 8,03 (dt, H-6'); 7,10 (dd, H-5');, 7,2-7,6 (m, übrige aromatische H); 1,65 (s, gem. $CH_3$),

ausgehend von 2-[4-Phenyl-5-(1-oxido-3-pyridyl]-2-methylpropionsäure und Triethylenglykol-monomethylether bzw. Triethylenglykol.
2-[4-Phenyl-5-(3-pyridyl)-oxazol-2-yl]-2-methyl-propionsäure-2[2-(2-hydroxyethoxy)-ethoxy]-ethylester (Oel),

NMR ($CDCl_3$): 8,85 (s, br, H-2'); 8,56 (d, br, H-6'); 7,87 (dt, H-4'); 7,64 (m, H-2",6"); 7,35-7,45 (übrige aromatische H); 7,18 (dd, H-5'); 1,75 s, gem.$CH_3$).

ausgehend von 2-[4-Phenyl-5-(3-pyridyl)-oxazol-2-yl]-2-methyl-propionsäure und Triethylenglykol.

Beispiel 23: Eine Lösung von 1,43 g Diethylenglykolmonochlorhydrin in 6 ml Hexamethylphosphorsäuretriamid wird bei 50-60° unter Rühren mit 3,46 g 2-[4-Phenyl-5-(3-pyridyl)-oxazol-2-yl]-2-methylpropionsäure-Natriumsalz versetzt. Die Mischung wird 4 Stunden bei 100° gerührt, abgekühlt und auf 50 ml Eiswasser gegossen. Das ausgeschiedene Oel wird mit 100 ml Ether extrahiert. Die Etherphase wird mit 20 ml Wasser, 20 ml 2-n. Kaliumhydrogencarbonatlösung und nochmals 20 ml Wasser gewaschen, über Magnesiumsulfat getrocknet und unter vermindertem Druck eingedampft. Den Rückstand chromatographiert man an 100 g Silicagel. Die Fraktionen 1-3, eluiert mit je 80 ml Chloroform, werden verworfen. Die Fraktionen 4-6, eluiert mit je 80 ml

Chloroform, werden vereinigt und unter vermindertem Druck zur Trockne eingedampft. Der Rückstand, der 2-[4-Phenyl-5-(1-oxido-3-pyridyl)-oxazol-2-yl]-2-methyl-propionsäure-[2-(2-hydroxyethoxy)-ethyl]-ester liegt als Oel vor.

NMR ($CDCl_3$): 8,39 (t, H-2'); 8,03 (dt, H-6'); 7,10 (dd, H-5');, 7,2-7,6 (m, übrige aromatische H); 1,65 (s, gem. $CH_3$).

Analog erhält man:
2-[4-Phenyl-5-(3-pyridyl)-oxazol-2-yl]-2-methyl-propionsäure-2-(2-hydroxyethoxy)-ethylester, (Oel),
NMR ($CDCl_3$): 8,85 (s, br, H-2'); 8,56 (d, br, H-6'); 7,87 (dt, H-4'); 7,64 (m, H-2",6"); 7,35-7,45 (m, übrige aromatische H); 7,18 (dd, H-5'); 1,75 (s, gem. $CH_3$),
ausgehend von 2-[4-Phenyl-5-(3-pyridyl)-oxazol-2-yl]-2-methyl-propionsäure-Natriumsalz und Diethylenglykol-monochlorhydrin.

Patentansprüche

1. Trisubstituierte Oxazaverbindungen der allgemeinen Formel

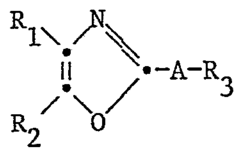

(I) ,

worin einer der Reste $R_1$ und $R_2$ Heteroaryl und der andere carbocyclisches Aryl oder Heteroaryl bedeutet und A einen zweiwertigen Kohlenwasserstoffrest darstellt und $R_3$ Carboxy, verestertes oder amidiertes Carboxy bedeutet, ihre Isomeren und ihre Salze.

2. Verbindungen der Formel (I) gemäss Anspruch 1, mit der Massgabe, dass dass $R_1$ bzw. $R_2$ von unsubstituiertem Thienyl und von unsubstituiertem Furyl verschieden ist.

3. Verbindungen der Formel (I) gemäss Anspruch 1, worin einer der Reste $R_1$ und $R_2$ einen 5- oder 6-gliedrigen, als Heteroatom(e) Stickstoff aufweisenden Heteroarylrest bedeutet, welcher jeweils unsubstituiert oder durch Halogen, Niederalkyl, Hydroxy, Niederalkoxy und/oder Niederalkanoyloxy substituiert sein kann und der andere unsubstituiertes oder durch Halogen, Niederalkyl, Hydroxy, Niederalkoxy, und/oder Niederalkanoyl substituiertes Phenyl oder einen 5- oder 6-gliedrigen, als Heteroatom(e) Stickstoff aufweisenden Heteroarylrest darstellt, der unsubstituiert oder durch Halogen, Niederalkyl, Hydroxy, Niederalkoxy und/oder Niederalkanoyloxy substituiert sein kann, bedeutet, A Niederalkylen, Niederalkyliden, Niederalkenylen, Niederalkenyliden, Cycloalkylen, Cycloalkyliden oder Cycloalkyl-niederalkyliden darstellt, und $R_3$ Carboxy, mit einem Niederalkanol, 3- bis 8-gliedrigem Cycloalkanol, Phenol, einem Hydroxypyridin, einem substituierten Phenol bzw. Hydroxypyridin verestertes Carboxy, Carbamoyl oder durch Hydroxy, Amino, Phenyl oder substituiertes Phenyl mono-substituiertes, durch Niederalkyl mono- oder disubstituiertes oder durch 4- bis 7-gliedriges Alkylen bzw. 3-Aza-, 3-Niederalkylaza-, 3-Oxa- oder 3-Thiaalkylen disubsti-

tuiertes Carbamoyl bedeutet, wobei ein Niederalkanol unsubstituiert oder durch Hydroxy, Mercapto, gegebenenfalls substituiertes Phenyl, Niederalkoxy, gegebenenfalls im Phenylteil substituiertes Phenylniederalkoxy, Niederalkylthio, gegebenenfalls im Phenylteil substituiertes Phenylniederalkylthio, Hydroxyniederalkoxy, Niederalkoxyniederalkoxy, gegebenenfalls im Phenylteil substituiertes Phenylniederalkoxyniederalkoxy, Hydroxyniederalkoxyniederalkoxy, Niederalkoxyniederalkoxyniederalkoxy, gegebenenfalls im Phenylteil substituiertes Phenylniederalkoxyniederalkoxyniederalkoxy, Carboxyniederalkoxy, Niederalkoxycarbonyl-niederalkoxy, gegebenenfalls substituiertes Phenyl aufweisendes Niederalkoxycarbonyl-niederalkoxy oder Niederalkanoyloxy substituiert sein kann und substituiertes Phenyl, Phenol bzw. Hydroxypyridin jeweils durch Niederalkyl, Niederalkoxy, Halogen und/oder Trifluormethyl substituiert sein kann, ihre Isomeren sowie ihre Salze.

4. Verbindungen der Formel (I) gemäss Anspruch 1, worin einer der Reste $R_1$ und $R_2$ Pyridyl oder 1-Oxido-pyridyl, bedeutet, die unsubstituiert und/oder jeweils durch Halogen, Hydroxy, Niederalkyl, Niederalkoxy und/oder Niederalkanoyloxy substituiert sein können, und der andere Phenyl, Pyridyl oder 1-Oxidopyridyl, die unsubstituiert und/oder jeweils durch Halogen, Hydroxy, Niederalkyl, Niederalkoxy und/oder Niederalkanoyloxy substituiert sein können, bedeutet, A Niederalkylen mit bis und mit 4 C-Atomen, wie Methylen, Niederalkyliden mit bis und mit 7 C-Atomen, wie 2,2-Propyliden, Niederalkenylen mit bis und mit 4 C-Atomen, wie 1,3-Propen-2-ylen, Niederalkenyliden mit bis und mit 7 C-Atomen, wie 1,1-Buten-3-yliden, 3- bis 8-gliedriges Cycloalkylen, wie Cyclopropylen, 3- bis 8-gliedriges Cycloalkyliden, wie Cyclopentyliden, oder Cycloalkyl-niederalkyliden mit bis und mit 7 C-Atomen im Alkylidenteil und mit einem 3- bis 8-gliedrigem Cycloalkylteil, wie 2-Cyclohexyl-1,1-ethyliden, bedeutet und $R_3$ Carboxy, mit einem Niederalkanol, einem 3- bis 8-gliedrigem Cycloalkanol, Phenol oder

einem substituierten Phenol verestertes Carboxy, Carbamoyl, N-Mono-, N,N-Diniederalkylcarbamoyl, Pyrrolidino-, Piperidino-, Morpholino-, Piperazino-, 4-Niederalkyl-piperazino-, Thiomorpholino-, Anilino- oder durch Niederalkyl, Niederalkoxy und/oder Halogen substituiertes Anilinocarbonyl darstellt, wobei das Niederalkanol unsubstituiert oder durch Hydroxy, Mercapto, Phenyl, substituiertes Phenyl, Niederalkoxy, Phenylniederalkoxy, im Phenylteil substituiertes Phenylniederalkoxy, Niederalkylthio, Phenylniederalkylthio, im Phenylteil substituiertes Phenylniederalkylthio, Hydroxyniederalkoxy, Niederalkoxyniederalkoxy, Phenylniederalkoxyniederalkoxy, im Phenylteil substituiertes Phenylniederalkoxyniederalkoxy, Hydroxyniederalkoxyniederalkoxy, Niederalkoxyniederalkoxyniederalkoxy, gegebenenfalls im Phenylteil substituiertes Phenylniederalkoxyniederalkoxy, Carboxyniederalkoxy, Niederalkoxycarbonylniederalkoxy, gegebenenfalls substituiertes Phenyl aufweisendes Niederalkoxycarbonyl-niederalkoxy oder Niederalkanoyloxy substituiert sein kann und substituiertes Phenol bzw. Phenyl jeweils durch Niederalkyl, Niederalkoxy, Halogen und/oder Trifluormethyl substituiert sein kann, ihre Isomeren sowie ihre Salze.

5. Verbindungen der Formel (I) gemäss Anspruch 1, worin einer der Reste $R_1$ und $R_2$ unsubstituiertes oder in zweiter Linie durch Halogen mit Atomnummer bis und mit 35, wie Chlor, Hydroxy, Niederalkyl mit bis und mit 4 C-Atomen, wie Methyl, und/oder Niederalkoxy mit bis und mit 4 C-Atomen, wie Methoxy, substituiertes Phenyl bedeutet und der andere Pyridyl, wie 3-Pyridyl, oder 1-Oxido-pyridyl, wie 1-Oxido-3-pyridyl, bedeutet, die jeweils unsubstituiert oder in zweiter Linie durch Halogen mit Atomnummer bis und mit 35, wie Chlor, Hydroxy und/oder Niederalkoxy mit bis und mit 4 C-Atomen, wie Methoxy, substituiert sein können, A Niederalkylen mit bis und mit 4 C-Atomen, wie Methylen, Niederalkyliden mit bis und mit 7 C-Atomen, wie 2,2-Propyliden, Niederalkenyliden mit bis und mit 7 C-Atomen, wie 1,1-Buten-3-yliden, oder 3- bis 8-gliedriges Cycloniederalkyliden, wie 1,1-Cyclopentyliden, darstellen und $R_3$ Carboxy, Niederalkoxycarbonyl mit bis und mit 5 C-Atomen, wie Ethoxycarbonyl, wobei das Niederalkoxycarbonyl durch

Niederalkanoyloxy mit bis und mit 5 C-Atomen, wie Pivaloyloxy, substituiert sein kann, Hydroxyniederalkoxy-niederalkoxycarbonyl, wie 2-(2-Hydroxyethoxy)-ethoxycarbonyl, Niederalkoxyniederalkoxy-niederalkoxycarbonyl, wie 2-(2-Methoxy-ethoxy)-ethoxycarbonyl, Hydroxyniederalkoxyniederalkoxy-niederalkoxycarbonyl, wie 2-[2-(2-Hydroxyethoxy)-ethoxy]-ethoxycarbonyl, oder Niederalkoxyniederalkoxyniederalkoxy-niederalkoxycarbonyl, wie 2-[2-(2-Methoxyethoxy)-ethoxy]-ethoxycarbonyl, jeweils mit bis und mit 4 C-Atomen im Niederalkoxy-Teil, bedeuten, ihre Isomeren sowie ihre Salze.

6. Verbindungen der Formel (I) gemäss Anspruch 1, worin einer der Reste $R_1$ und $R_2$ unsubstituiertes oder in zweiter Linie durch Halogen mit Atomnummer bis und mit 35, wie Chlor, Hydroxy oder Niederalkoxy mit bis und mit 4 C-Atomen, wie Methoxy, substituiertes Phenyl bedeutet und der andere Pyridyl, wie 3- oder 4-Pyridyl, oder 1-Oxidopyridyl, wie 1-Oxido-3-pyridyl oder 1-Oxido-4-pyridyl, darstellt, A Niederalkyliden mit bis und mit 4 C-Atomen, wie 2,2-Propyliden, bedeutet, und $R_3$ Niederalkoxycarbonyl mit bis und mit 5 C-Atomen, wie Ethoxycarbonyl, darstellt, ihre Isomeren sowie ihre Salze.

7. Verbindungen der Formel (I) gemäss Anspruch 1, worin $R_1$ Phenyl bedeutet, $R_2$ 1-Oxidopyridyl, wie 1-Oxido-3-pyridyl, darstellt, A 2,2-Propyliden bedeutet und $R_3$ Niederalkoxycarbonyl mit bis und mit 5 C-Atomen, wie Ethoxycarbonyl, darstellt, ihre Isomeren sowie ihre Salze.

8. 2-[4-Phenyl-5-(3-pyridyl)-oxazol-2-yl]-2-methyl-propionsäureethylester oder ein Salz davon.

9. 2-[4-Phenyl-5-(3-pyridyl)-oxazol-2-yl]-propionsäureethylester oder ein Salz davon.

10. 2-[4-Phenyl-5-(3-pyridyl)-oxazol-2-yl]-essigsäureethylester oder ein Salz davon.

11. 2-[4-Phenyl-5-(1-oxido-3-pyridyl)-oxazol-2-yl]-2-methyl-propionsäureethylester.

12. 2-[4-(4-Phenyl-5-(1-oxido-3-pyridyl)-oxazol-2-yl]-propionsäure-ethylester.

13. 2-[4-Phenyl-5-(1-oxido-3-pyridyl)-oxazol-2-yl]-essigsäureethylester.

14. 2-[4-Phenyl-5-(1-oxido-3-pyridyl)-oxazol-2-yl]-2-methyl-propionsäure oder ein Salz davon.

15. 2-[4-Phenyl-5-(1-oxido-3-pyridyl)-oxazol-2-yl]-propionsäure oder ein Salz davon.

16. 2-[4-Phenyl-5-(1-oxido-3-pyridyl)-oxazol-2-yl]-essigsäure oder ein Salz davon.

17. 2-[4-Phenyl-5-(3-pyridyl)-oxazol-2-yl]-2-methyl-propionsäure.

18. 2-[4-(3-Pyridyl)-5-phenyl-oxazol-2-yl]-2-methyl-propionsäure-ethylester.

19. 2-[4-(3-Pyridyl)-5-phenyl-oxazol-2-yl]-propionsäureethylester.

20. 2-[4-(3-Pyridyl)-5-phenyl-oxazol-2-yl]-essigsäureethylester.

21. 2-[4-(1-Oxido-3-pyridyl)-5-phenyl-oxazol-2-yl]-2-methyl-propionsäureethylester.

22. 2-[4-(1-Oxido-3-pyridyl)-5-phenyl-oxazol-2-yl]-propionsäureethylester.

23. 2-[4-(1-Oxido-3-pyridyl)-5-phenyl-oxazol-2-yl]-essigsäureethylester.

24. 2-[4-Phenyl-5(4)-(3-pyridyl)-oxazol-2-yl]-2-methyl-propionsäure-tert.-butylester oder ein Salz davon.

25. 2-[4-(3-Pyridyl)-5-phenyl-oxazol-2-yl]-2-methyl-propionsäure-tert.-butylester oder ein Salz davon.

26. 2-[4-Phenyl-5-(1-oxido-3-pyridyl)-oxazol-2-yl]-2-methyl-propionsäure-tert.-butylester.

27. 2-[4-(1-Oxido-3-pyridyl)-5-phenyl-oxazol-2-yl]-2-methyl-propionsäure-tert.-butylester.

28. 2-[4-Phenyl-5-(3-pyridyl)-oxazol-2-yl]-2-methyl-propionsäure-2-[2-(2-methoxyethoxy)-ethoxy]-ethylester oder ein Salz davon.

29. 2-[4-Phenyl-5-(1-oxido-3-pyridyl)-oxazol-2-yl]-2-methyl-propionsäure-2-[2-(2-methoxyethoxy)-ethoxy]-ethylester.

30. 2-[4-Phenyl-5-(3-pyridyl)-oxazol-2-yl]-2-methyl-propionsäure-2-(2-hydroxyethoxy)-ethylester oder ein Salz davon.

31. 2-[4-Phenyl-5-(1-oxido-3-pyridyl)-oxazol-2-yl)-2-methyl-propionsäure-2-(2-hydroxyethoxy)-ethylester.

32. 2-[4-Phenyl-5-(3-pyridyl)-oxazol-2-yl]-2-methyl-propionsäure-2-[2-(2-hydroxyethoxyl)-ethoxy]-ethylester oder ein Salz davon.

33. 2-[4-Phenyl-5-(1-oxido-3-pyridyl)-oxazol-2-yl]-2-methyl-propionsäure-2-[2-(2-hydroxyethoxy)-ethoxy]-ethylester.

34. Verbindung gemäss einem der Ansprüche 1 bis 33 mit Wirkung als externes Hautphlogistatikum.

35. Pharmazeutische Präparate, enthaltend mindestens eine Verbindung gemäss einem der Ansprüche 1 bis 34 neben üblichen Hilfs- und Trägerstoffen.

36. Verfahren zur Herstellung von Verbindungen der Formel (I) gemäss Anspruch 1, dadurch gekennzeichnet, dass man eine Verbindung der Formel

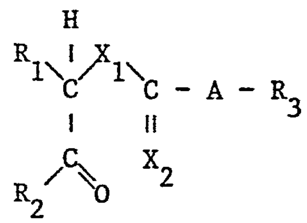

(II) ,

ein Tautomeres und/oder Salz davon, worin einer der Reste $X_1$ und $X_2$ gegebenenfalls substituiertes Imino und der andere Oxy oder gegebenenfalls substituiertes Imino bedeutet, cyclisiert oder, worin $X_1$ Oxy und $X_2$ Oxo darstellen, unter Kondensation cyclisiert oder eine Verbindung der Formel

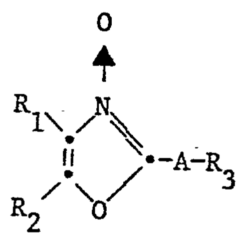

(III)

oder ein Salz davon zu einer Verbindung der Formel I reduziert oder in einer Verbindung der Formel

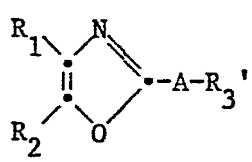

(IV) ,

worin $R_3$' einen im $R_3$ überführbaren Rest bedeutet, den Rest $R_3$' in einen Rest $R_3$ überführt und gewünschtenfalls eine verfahrensgemäss erhältliche freie Verbindung der Formel (I) in eine andere freie Verbindung überführt, eine verfahrensgemäss erhältliche freie Verbindung in ein Salz oder ein verfahrensgemäss erhältliches Salz in ein anderes Salz oder in eine freie Verbindung überführt und, wenn erwünscht, ein verfahrensgemäss erhältliches Isomerengemisch in seine Komponenten auftrennt.

37. Verfahren gemäss Anspruch 36, dadurch gekennzeichnet, dass man von einer auf irgendeiner Stufe des Verfahrens als Zwischenstufe erhältlichen Verbindung ausgeht und die fehlenden Schritte durchführt oder einen Ausgangsstoff in Form eines Salzes, Isomeren und/oder Racemates bzw. Antipoden verwendet oder unter den Reaktionsbedingungen bildet.

38. Verfahren nach einem der Ansprüche 36 und 37, dadurch gekennzeichnet, dass man eine Verbindung der Formel

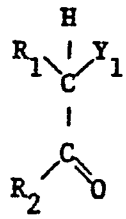

(IIa) ,

ein Tautomeres und/oder Salz davon, worin $Y_1$ gegebenenfalls funktionell abgewandeltes Hydroxy bedeutet, mit einer Verbindung der Formel $R_3$-A-$Y_2$ (IIb), worin $Y_2$ mindestens ein Stickstoffatom aufweisender funktionell abgewandeltes Carboxy darstellt, oder einem Salz davon oder eine Verbindung der Formel (IIa), ein Tautomeres und/oder Salz davon, worin $Y_1$ Amino bedeutet, mit einer Verbindung der Formel (IIb), worin $Y_2$ gegebenenfalls funktionell abgewandeltes Carboxy darstellt, oder einem Salz davon, gegebenenfalls in Gegenwart eines Kondensationsmittels, umsetzt oder Verbindungen der Formel

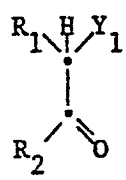

(IIa),

worin $Y_1$ gegebenenfalls reaktionsfähig verestertes Hydroxy bedeutet, mit Carbonsäuren der Formel $R_3$-A-COOH (IIc), deren Salze oder deren funktionelle Derivate und mit Ammoniak umsetzt, oder Verbindungen der Formel

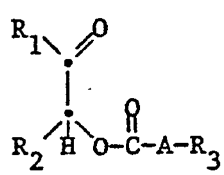

(IId),

welche durch Kondensation von Verbindungen der Formel (IIa) mit gegebenenfalls funktionellen Derivaten von Verbindungen der Formel (IIc) erhältlich sind, mit Ammoniak umsetzt.

39. Verfahren nach einem der Ansprüche 36 und 37, dadurch gekennzeichnet, dass man von Verbindungen der Formel (IV) oder deren Salzen ausgeht , worin $R_3$' einen solvoltytisch in $R_3$ überführbaren Rest bedeutet, $R_3$' durch Solvolyse in $R_3$ überführt, oder worin $R_3$' einen oxidativ in $R_3$ überführbaren Rest darstellt, $R_3$' durch Oxidation in $R_3$ überführt.

40. Verfahren zur Herstellung pharmazeutischer Präparate, dadurch gekennzeichnet, dass man mindestens eine Verbindung gemäss einem der Ansprüche 1 bis 34, gegebenenfalls unter Verwendung üblicher Hilfs- und Trägerstoffe, in eine geeignete Applikationsform überführt.

41. Verbindung gemäss einem der Ansprüche 1 bis 34 zur therapeutischen Behandlung des menschlichen oder tierischen Körpers.

42. Verwendung von Verbindungen gemäss einem der Ansprüche 1 bis 34 in einem Verfahren zur Behandlung entzündlicher Dermatosen.

43. Die in den Beispielen 1 bis 13 und 20 bis 27 genannten neuen Verbindungen.

44. Das Verfahren der Beispiele 1 bis 27 und die danach erhältlichen neuen Verbindungen.

45. Die im Verfahren gemäss einem der Ansprüche 36 bis 39 verwendeten neuen Ausgangsstoffe und/oder Zwischenprodukte.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0084756

Nummer der Anmeldung

EP 82 81 0027

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X,Y | US - A - 3 578 671 (K. BROWN)<br>* das ganze Dokument *<br>-- | 1-44 | C 07 D 413/04<br>A 61 K 31/44<br>//C 07 D 213/50<br>213/53 |
| X,Y | GB - A - 1 542 315 (J. WYETH)<br>* das ganze Dokument *<br>-- | 1-44 | |
| Y | CH - A - 561 718 (CIBA-GEIGY)<br>* das ganze Dokument *<br>-- | 1-44 | |
| E | EP - A - 0 045 081 (CIBA-GEIGY)<br>* das ganze Dokument *<br>---- | 1-34,<br>36-41,<br>43,44 | RECHERCHIERTE SACHGEBIETE (Int. Cl. 3)<br>C 07 D 413/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-42.

Unvollständig recherchierte Patentansprüche:

Nicht recherchierte Patentansprüche: 43,44.

Grund für die Beschränkung der Recherche: Regel 29.6 EPU

| Recherchenort | Abschlußdatum der Recherche | Prüfer |
|---|---|---|
| DEN HAAG | 06-09-1982 | ALLARD |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

0084756

Europäisches Patentamt

| | GEBÜHRENPFLICHTIGE PATENTANSPRÜCHE |
|---|---|

Die vorliegende europäische Patentanmeldung enthielt bei ihrer Einreichung mehr als zehn Patentansprüche.

☐ Alle Anspruchsgebühren wurden innerhalb der vorgeschriebenen Frist entrichtet. Der vorliegende europäische Recherchenbericht wurde für alle Patentansprüche erstellt.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt für die Anspruchsgebühren entrichtet wurden,

nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

| X | MANGELNDE EINHEITLICHKEIT DER ERFINDUNG |
|---|---|

Nach Auffassung der Recherchenabteilung entspricht die vorliegende europäische Patentanmeldung nicht den Anforderungen an die Einheitlichkeit der Erfindung; sie enthält mehrere Erfindungen oder Gruppen von Erfindungen, nämlich:

1. Patentansprüche 1-42: Oxazolderivate, ihre Herstellung und Anwendung
2. Patentanspruch 45 : Ausgangs- und Zwischenstoffe, soweit nicht unter Anspruch 1 fallend

☐ Alle weiteren Recherchengebühren wurden innerhalb der gesetzten Frist entrichtet. Der vorliegende europäische Recherchenbericht wurde für alle Patentansprüche erstellt.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind,

nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen,

nämlich Patentansprüche: 1-42